(19) **Europäisches Patentamt**
European Patent Office
Office européen des brevets

(11) **EP 3 998 941 B1**

(12) **EUROPÄISCHE PATENTSCHRIFT**

(45) Veröffentlichungstag und Bekanntmachung des Hinweises auf die Patenterteilung:
**29.10.2025 Patentblatt 2025/44**

(21) Anmeldenummer: **20742230.4**

(22) Anmeldetag: **15.07.2020**

(51) Internationale Patentklassifikation (IPC):
*A61B 5/091* (2006.01)   *A61M 16/00* (2006.01)

(52) Gemeinsame Patentklassifikation (CPC):
(C-Sets verfügbar)
**A61B 5/091; A61M 16/024; A61M 16/1005;**
**A61M 16/125;** A61M 16/0066; A61M 16/04;
A61M 16/0808; A61M 16/0833; A61M 16/0858;
A61M 16/0883; A61M 16/107; A61M 16/16;
A61M 16/204; A61M 16/205; A61M 2016/0036;
(Forts.)

(86) Internationale Anmeldenummer:
**PCT/EP2020/069983**

(87) Internationale Veröffentlichungsnummer:
**WO 2021/009215 (21.01.2021 Gazette 2021/03)**

(54) **BEATMUNGSVORRICHTUNG ZUR AUSFÜHRUNG EINES VERFAHRENS ZUR ERMITTLUNG EINER FUNKTIONALEN RESTKAPAZITÄT EINER PATIENTENLUNGE**

VENTILATOR FOR CARRYING OUT A METHOD FOR DETERMINING THE FUNCTIONAL RESIDUAL CAPACITY OF A PATIENT'S LUNG

DISPOSITIF RESPIRATOIRE PERMETTANT DE METTRE EN OEUVRE UN PROCÉDÉ PERMETTANT DE DÉTERMINER UNE CAPACITÉ RÉSIDUELLE FONCTIONNELLE D'UN POUMON D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.07.2019 DE 102019119575**

(43) Veröffentlichungstag der Anmeldung:
**25.05.2022 Patentblatt 2022/21**

(73) Patentinhaber: **Hamilton Medical AG**
**7402 Bonaduz (CH)**

(72) Erfinder:
• **LAUBSCHER, Thomas**
**7403 Rhäzüns (CH)**
• **SCHRANZ, Christoph**
**7306 Fläsch (CH)**
• **NOVOTNI, Dominik**
**7000 Chur (CH)**
• **REIDT, Sascha**
**7206 Igis (CH)**

(74) Vertreter: **Ruttensperger Lachnit Trossin Gomoll Patent- und Rechtsanwälte**
**PartG mbB**
**Arnulfstraße 58**
**80335 München (DE)**

(56) Entgegenhaltungen:
**EP-A1- 0 653 183   EP-B1- 0 653 183**
**WO-A1-00/44280   WO-A1-2007/065475**

• **OLEGAARD C ET AL: "ESTIMATION OF FUNCTIONAL RESIDUAL CAPACITY AT THE BEDSIDE USING STANDARD MONITORING EQUIPMENT: A MODIFIED NITROGEN WASHOUT/WASHIN TECHNIQUE REQUIRING A SMALL CHANGE OF THE INSPIRED OXYGEN FRACTION", ANESTHESIA AND ANALGESIA, WILLIAMS AND WILKINS, BALTIMORE, MD, US, vol. 101, no. 1, 1 January 2005 (2005-01-01), pages 206 - 212, XP009063847, ISSN: 0003-2999, DOI: 10.1213/01.ANE.0000165823.90368.55**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**(Forts. nächste Seite)**

(52) Gemeinsame Patentklassifikation (CPC): (Forts.)
A61M 2016/1025; A61M 2016/103;
A61M 2205/3306; A61M 2205/3358;
A61M 2205/502; A61M 2230/40; A61M 2230/432;
A61M 2230/435

C-Sets
A61M 2230/432, A61M 2230/005;
A61M 2230/435, A61M 2230/005

**Beschreibung**

Die vorliegende Erfindung betrifft eine Beatmungsvorrichtung, welche sowohl zur wenigstens teilweisen künstlichen Beatmung von lebenden Patienten als auch zur Durchführung eines Verfahrens zur Ermittlung einer funktionalen Restkapazität einer Patientenlunge ausgebildet ist, wobei die Beatmungsvorrichtung umfasst:

- eine erste Atemgasquelle, welche eine erste inspiratorische Atemgaskomponente mit einer ersten Fraktion eines metabolisch-inerten Gases bereitstellt,
- eine zweite Atemgasquelle, welche eine zweite inspiratorische Atemgaskomponente mit einer von der ersten verschiedenen zweiten Fraktion des metabolisch-inerten Gases bereitstellt,
- eine veränderlich einstellbare Mischvorrichtung zur Bildung eines inspiratorischen Atemgases mit veränderlichem Anteil an metabolisch-inertem Gas aus der ersten oder/und der zweiten inspiratorischen Atemgaskomponente,
- eine Beatmungsleitungsanordnung zur Förderung des inspiratorischen Atemgases zu einem patientenseitigen Atemgasauslass und zur Förderung von exspiratorischem Atemgas von einem patientenseitigen Atemgaseinlass weg von dem Atemgaseinlass,
- eine Steuerventilanordnung, umfassend ein Inspirationsventil und ein Exspirationsventil,
- eine Druckveränderungsvorrichtung zur Veränderung wenigstens des inspiratorischen Atemgases in der Beatmungsleitungsanordnung,
- eine Flusssensoranordnung zur Erfassung wenigstens des inspiratorischen Atemgasflusses,
- eine Gaskomponenten-Sensoranordnung zur mittelbaren oder unmittelbaren Erfassung des Anteils des metabolisch-inerten Gases am inspiratorischen und am exspiratorischen Atemgas,
- eine Steuervorrichtung, welche zur Steuerung der Steuerventilanordnung und der Druckveränderungsvorrichtung ausgebildet ist und welche signalübertragungsmäßig mit der Flusssensoranordnung und der Gaskomponenten-Sensoranordnung zur Übertragung von jeweiligen Erfassungssignalen an die Steuervorrichtung verbunden ist.

Das Verfahren zur Ermittlung einer funktionalen Restkapazität (FRC) einer Patientenlunge, zu dessen Durchführung die Beatmungsvorrichtung ausgebildet ist, umfasst die folgenden Schritte:

- Zuführen eines ersten inspiratorischen Atemgases mit einem ersten Anteil an einem metabolisch-inerten Gas während einer ersten zeitlichen Zufuhrphase,
- nach der ersten Zufuhrphase: Zuführen eines vom ersten verschiedenen zweiten inspiratorischen Atemgases mit einem vom ersten verschiedenen zweiten Anteil an dem metabolisch-inerten Gas während einer zweiten zeitlichen Zufuhrphase,
- Ermitteln einer während der zweiten Zufuhrphase auftretenden Mengendifferenz, welche für einen Ermittlungszeitraum eine Unterschiedsmenge repräsentiert zwischen einer Menge an inspiratorischem und einer Menge an exspiratorischem metabolisch-inerten Gas, wobei der Ermittlungszeitraum nicht nach der zweiten Zufuhrphase endet,
- Ermitteln der funktionalen Restkapazität unter Berücksichtigung der Mengendifferenz und einer Anteilsdifferenz zwischen einer ersten Anteilsgröße, welche den ersten Anteil an dem metabolisch-inerten Gas im ersten inspiratorischen Arbeitsgas repräsentiert und einer zweiten Anteilsgröße, welche den zweiten Anteil an dem metabolisch-inerten Gas im zweiten inspiratorischen Arbeitsgas repräsentiert.

Das Verfahren als solches ist nicht Gegenstand der beanspruchten Erfindung.

Nachdem eingangs klargestellt wurde, dass es sich bei den Zufuhrphasen um zeitliche Zufuhrphasen handelt, sind die Zufuhrphasen nachfolgend ohne den Zusatz "zeitlich" bezeichnet.

Ein derartiges Verfahren ist bekannt aus Olegård, C. et al.: "Estimation of Functional Residual Capacity at the Bedside Using Standard Monitoring Equipment: A Modified Nitrogen Washout/Washin Technique Requiring a Small Change of the Inspired Oxygen Fraction", in International Anesthesia Research Society: "Anesthesia & Analgesia", 2005 (101), Seiten 206 - 212. Dieses Verfahren wird in der Fachliteratur und in der vorliegenden Anmeldung als "Verfahren nach Olegård" bezeichnet.

Derartige Verfahren zur Ermittlung der funktionalen Restkapazität, oder kurz FRC für "Functional Residual Capacity", sind allgemein als Auswaschverfahren bekannt, im Gegensatz zu ebenfalls existierenden Dilutionsverfahren und zur bekannten Bodyplethysmographie, da diesen Verfahren eine Beobachtung des Auswaschens des metabolisch-inerten Gases aus der Patientenlunge nach Änderung der Zusammensetzung inspiratorischen Atemgases zugrunde liegt.

Auswaschverfahren sind ebenfalls aus Wauer, H. J. et al.: "FRC-Messung bei beatmeten Intensivpatienten - Eine Standortbestimmung", in Springer Verlag: "Der Anaesthesist", 1998 (47), Seiten 844 - 855, bekannt.

Ein weiteres Auswaschverfahren und eine Vorrichtung hierfür sind aus der US 7,530,353 B2 bekannt.

Die Ermittlung einer FRC durch Auswaschverfahren beruht auf der Ermittlung der Menge an ausgewaschenem metabolisch-inertem Gas während einer Auswaschung, sowie auf der Ermittlung des Unterschiedes an Anteilen an metabolisch-inerten Gas im inspiratorischen Atemgas vor und während des Auswaschvorgangs. Als Auswaschvorgang wird im Rahmen der vorliegenden Anmeldung wenigstens jene Zeitspanne verstanden, welche reicht von einem Zeitpunkt unmittelbar ab Beginn der zweiten Zufuhrphase, bis zu dem Zeitpunkt in der zweiten Zufuhrphase, in welchem die Patientenlunge und damit das aus ihr abströmende exspiratorische Atemgas während einer Beatmung mit dem zweiten inspiratorischen Atemgas über mehrere Atemhübe hinweg wieder einen im Wesentlichen konstanten Anteil an dem metabolisch-inerten Gas zeigt. Der Auswaschvorgang und damit der Ermittlungszeitraum kann kürzer als die zweite Zufuhrphase sein. Er ist jedoch nicht länger als die zweite Zufuhrphase.

Bedingung für den Beginn der zweiten Zufuhrphase ist, dass das exspiratorische Atemgas in der ersten Zufuhrphase einen im Wesentlichen konstanten Anteil an dem metabolisch-inerten Gas aufweist. Andernfalls kann das Auswaschverfahren zwar durchgeführt werden, führt jedoch zu einem ungenauen bzw. falschen Ergebnis.

Die oben genannten Veröffentlichungen des Standes der Technik unterscheiden sich im Wesentlichen in der Berechnung der Menge an ausgewaschenem metabolisch-inertem Gas. Diese Menge an ausgewaschenem metabolisch-inertem Gas wird in der vorliegenden Anmeldung repräsentiert durch die Mengendifferenz zwischen einer Menge an inspiratorischem und einer Menge an exspiratorischem metabolisch-inertem Gas. Während bei dem Verfahren nach Olegård für jeden Atemhub eine tidale Mengendifferenz zwischen den Mengen an metabolisch-inertem Gas im inspiratorischen und im exspiratorischen Atemgas gebildet und über die Dauer des Auswaschvorgangs hinweg aufsummiert wird, ermitteln Wauer et al. sowie die US 7,530,353 B2 die Menge an ausgewaschenem metabolisch-inertem Gas durch Ermittlung der tidalen Menge an metabolisch-inertem Gas im exspiratorischen Atemgas unmittelbar vor Beginn der zweiten Zufuhrphase als einer Basismenge, sowie durch Aufsummierung von tidalen Differenzwerten, welche gebildet werden durch Subtraktion der Basismenge von der jeweiligen tidalen Menge an metabolisch-inertem Gas im exspiratorischen Atemgas, über die Atemhübe des Auswaschvorgangs hinweg.

Ein gewisser Vorteil der letztgenannten Berechnungsmethode liegt darin, dass ihr Algorithmus unabhängig vom inspiratorischen Atemgas ist. Sie geht von der Annahme aus, dass jeglicher Unterschied in den pro Atemhub ausgeatmeten tidalen Mengen an metabolisch-inertem Gas gegenüber der ausgeatmeten tidalen Menge vor Beginn der zweiten Zufuhrphase alleine durch die Auswaschung von zu Beginn der zweiten Zufuhrphase noch in der Patientenlunge vorhandenem metabolisch-inertem Gas zurückgeht. Diese Annahme ist jedoch bestenfalls unter idealen Laborbedingungen gerechtfertigt, in welchen beispielsweise inspiratorisches Atemgas leckagefrei dem Patienten zugeführt werden kann. Weichen die tatsächlichen Zufuhrbedingungen jedoch von idealen Laborbedingungen ab, was selbst unter günstigen Bedingungen im Labor der Fall ist, nimmt die Ungenauigkeit der letztgenannten Berechnungsmethode zu.

Das Verfahren nach Olegård geht dagegen von der Annahme aus, dass die Differenz zwischen der eingeatmeten und der ausgeatmeten tidalen Menge an metabolisch-inertem Gas allein auf einer Auswaschung von zu Beginn der zweiten Zufuhrphase noch in der Patientenlunge vorhandenem metabolisch-inertem Gas beruht. Diese Annahme ist grundsätzlich vernünftig, da der Begriff "metabolisch-inert" gerade anzeigt, dass eine so bezeichnete Substanz im Patientenkörper nicht verstoffwechselt wird, sodass zunächst kein Grund ersichtlich ist, warum im exspiratorischen und im inspiratorischen Atemgas nicht gleiche Mengen an metabolisch-inertem Gas enthalten sein sollen. Da das Verfahren nach Olegård auch das inspiratorische Atemgas berücksichtigt, liefert es theoretisch genauere Ergebnisse als die zuvor besprochene Berechnungsmethode. Allerdings beschreibt Olegård in dem genannten Artikel die Probleme bei der Erfassung der Menge an metabolisch-inertem Gas im inspiratorischen Atemgas, weshalb im Verfahren nach Olegård die Werte des inspiratorischen Atemgases nicht messtechnisch erfasst, sondern auf Grundlage einer Haldane-Transformation aus den messtechnisch allein erfassten Werten des exspiratorischen Atemgases abgeleitet werden. Dennoch liefert das Verfahren nach Olegård genauere Ergebnisse als die oben genannten anderen Verfahren.

Zielsetzung der vorliegenden Erfindung ist, die Ermittlung der funktionalen Restkapazität einer Patientenlunge im normalen klinischen Alltag, d. h. ohne besondere Laborumgebung oder Laborbedingungen zu ermöglichen. Insbesondere soll die Ermittlung der funktionalen Restkapazität mit der Beatmungsvorrichtung während eines Beatmungsbetriebs zur wenigstens teilweisen künstlichen Beatmung eines Patienten möglich sein.

Entsprechende Versuche haben gezeigt, dass bei Anwendung eines Auswaschverfahrens in klinischen Alltagssituationen die tidale Mengendifferenz zwischen dem Anteil an metabolisch-inertem Gas im inspiratorischen Atemgas und dem Anteil an metabolisch-inertem Gas im exspiratorischen Atemgas auch lange nach Beginn der zweiten Zufuhrphase nicht zu 0 wird, sondern sich einem Offset-Wert annähert bzw. diesen annimmt. Hierfür werden nach derzeitigen, noch unvollständigen Kenntnissen mehrere Ursachen verantwortlich gemacht: zum einen sind an den Beatmungsleitungsanordnungen, mittels welcher inspiratorisches Atemgas den Patienten zugeführt und exspiratorischen Atemgas vom Patienten weggeführt wird, Leckagen vorhanden, welche das Atemgas in seiner Zusammensetzung verändern können. Zum anderen können Messfehler in der Flussmessung des Atemgasflusses auftreten, etwa durch asymmetrische Anströmung eines Strömungswiderstands eines Differenzdruck-Flusssensors oder/und durch asymmetrische Befeuchtung oder/und Tröpfchenbildung im Differenzdruck-Flusssensor usw.

Aus der WO 00/44280 A1 ist ein eingangs genanntes Verfahren zur Ermittlung einer funktionalen Restkapazität einer Lunge bekannt, in welchem der zuvor genannte betragsmäßige Unterschied zwischen mengenmäßig während des Ermittlungszeitraums eingeatmetem metabolisch-inertem Gas und ausgeatmetem metabolisch-inertem Gas durch einen zur ausgeatmeten Menge hinzu multiplizierten Korrekturfaktor zwangsweise betragsmäßig zu 0 gemacht wird. Der Korrekturfaktor wird durch Regressionsverfahren, wie beispielsweise die Methode der kleinsten Fehlerquadrate, ermittelt.

Die Annahmen gehen dahin, dass zum einen die Beatmungsvorrichtung als solche und zum anderen die mit der jeweiligen Beatmungsvorrichtung geschaffene Beatmungssituation jeweils einen Fehlereinfluss auf die Ermittlung der FRC haben können.

Es ist daher Aufgabe der vorliegenden Erfindung, die eingangs genannte Beatmungsvorrichtung durch Weiterentwicklung des Verfahrens derart zu verbessern, dass das Verfahren mit ausreichend hoher Genauigkeit im Ergebnis auch in klinischer Umgebung ohne Laborqualität ausgeführt werden kann.

Diese Aufgabe wird erfindungsgemäß dadurch gelöst, dass das Verfahren, zu dessen Durchführung die Beatmungsvorrichtung ausgebildet ist, folgende weitere Schritte umfasst:

- Ermitteln einer Basisdifferenz, welche eine Differenz zwischen einer tidalen Menge an inspiratorischem metabolisch-inerten Gas und einer tidalen Menge an exspiratorischem metabolisch-inerten Gas in der ersten oder/und der zweiten Zufuhrphase repräsentiert,

wobei das Ermitteln der funktionalen Restkapazität auf Grundlage einer korrigierten Mengendifferenz und der Anteilsdifferenz erfolgt, wobei die korrigierte Mengendifferenz durch Berücksichtigung der Basisdifferenz bei der Ermittlung der Mengendifferenz gebildet wird.

Die geschilderte Basisdifferenz ist eine auf einen einzelnen Atemhub bezogene Größe und repräsentiert ein Maß für den oben beschriebenen Offset-Wert, der an einer erfindungsgemäßen Beatmungsvorrichtung, mit welcher das Verfahren ausgeführt wird, selbst lange nach Beginn der zweiten Zufuhrphase bestehen kann. Somit kann der weder bekannte noch vorhersagbare Einfluss der Beatmungsvorrichtung oder/und der Einfluss der jeweils herrschenden Beatmungssituation quantifiziert und bei der Berechnung der FRC berücksichtigt werden. Der als Ergebnis des Verfahrens ermittelte Wert der FRC kann somit auch in solchen Umgebungen zuverlässig mit sehr guter Genauigkeit erhalten werden, bei denen sich die Umgebungsbedingungen ändern oder/und die Umgebungsbedingungen teilweise unbekannt sind.

Der Ermittlungszeitraum kann die gesamte zweite Zufuhrphase sein oder kann ein zeitlich kürzerer Zeitraum als die zweite Zufuhrphase sein. Beispielsweise kann der Ermittlungszeitraum enden, wenn sich Differenzenwerte, welche Tidalmengendifferenzen zwischen der tidalen Menge an metabolisch-inertem Gas im inspiratorischen Atemgas und der tidalen Menge an metabolisch-inertem Gas im exspiratorischen Atemgas repräsentieren, für aufeinanderfolgende Atemhübe betragsmäßig um weniger als einen vorbestimmten Differenzen-Schwellenwert unterscheiden. Dann ist für die zweite Zufuhrphase ein Zustand erreicht, bei welchem ein durch den Differenzen-Schwellenwert bestimmtes ausreichendes Gleichgewicht zwischen den Mengen an metabolisch-inertem Gas im inspiratorischen und im exspiratorischen Atemgas erreicht ist. Der Ermittlungszeitraum entspricht dann faktisch der Dauer eines Auswasch- oder auch eines Einspülvorgangs. Ein Einspülvorgang entspricht einem Auswaschvorgang mit dem einzigen Unterschied, dass beim Auswaschvorgang der Anteil an metalisch-inertem Gas im ersten inspiratorischen Atemgas höher ist als im zweiten inspiratorischen Atemgas, während es sich bei einem Einspülvorgang genau umgekehrt verhält.

Bevorzugt beginnt der Ermittlungszeitraum gleichzeitig mit der zweiten Zufuhrphase.

Um eine möglichst genaue Ermittlung der FRC zu ermöglichen, sollte der Übergang von der ersten zur zweiten Zufuhrphase möglichst kurz sein, möglichst kürzer als ein Atemhub. Besonders bevorzugt findet der Übergang von der ersten zur zweiten Zufuhrphase während einer Exspirationsphase des Patienten statt, sodass der Patient während der der Exspirationsphase unmittelbar vorhergehenden Inspirationsphase noch das erste inspiratorische Atemgas verabreicht erhält und während der der Exspirationsphase unmittelbar nachfolgenden Inspirationsphase bereits das zweite inspiratorische Atemgas verabreicht erhält.

Die Tidalmengendifferenz eines n-ten Atemzugs nach Beginn der i-ten Zufuhrphase, in welcher er stattgefunden hat, lässt sich formelmäßig darstellen als:

$$^{iZp}\Delta v(n)^{tid}_{miG} = {}^{iZp}_{in}v(n)^{tid}_{miG} - {}^{iZp}_{ex}v(n)^{tid}_{miG} \qquad\qquad \text{(Gl. 1)}$$

mit "tid" für "tidal", "miG" für metabolisch-inertes Gas und mit "iZp" als Kennzeichnung für die Zufuhrphase, wobei i = 1 für die erste Zufuhrphase und mit i = 2 für die zweite Zufuhrphase ist, wobei weiter $^{iZp}\Delta v(n)^{tid}_{miG}$ die Tidalmengendifferenz des n-ten Atemzugs der i-ten Zufuhrphase bezeichnet, $^{iZp}_{in}v(n)^{tid}_{miG}$ die tidale Menge an metabolisch-inertem Gas im inspiratorischen Atemgas im n-ten Atemhub der i-ten Zufuhrphase bezeichnet, und wobei $_{ex}^{iZp}v(n)_{miG}^{tid}$ die tidale Menge an metabolisch-inertem Gas im exspiratorischen Atemgas im n-ten Atemhub der i-ten Zufuhrphase bezeichnet. Für jede Zufuhrphase beginnt n gemäß der vorliegenden Anmeldung erneut von 1 ab sich inkrementell erhöhend zu laufen.

Zur Ermittlung der Tidalmengen $_{in}^{iZp}v(n)_{miG}^{tid}$ an inspiratorischem metabolisch-inertem Gas und $_{ex}^{iZp}v(n)_{miG}^{tid}$ exspiratorischem metabolisch-inertem Gas bestehen mehrere Möglichkeiten. Zum einen besteht die Möglichkeit, diese Werte zu ermitteln, wie dies im Stand der Technik aus der oben zitierten Olegärd-Veröffentlichung bekannt ist.

Olegård ermittelt gemäß der an sich bekannten Formel von Bohr eine tidale Menge an exspiratorischem alveolarem Atemgas aus einer über einen vorbestimmten Zeitraum ermittelten exspiratorischen Menge an $CO_2$, einem diesem Zeitraum zugeordneten endexspiratorischen bzw. endtidalen $CO_2$-Anteilswert, welcher jeweils den endexspiratorischen bzw. endtidalen Anteil an $CO_2$ in den Atemhüben des vorbestimmten Zeitraums angibt, und der Anzahl an Atemhüben im vorbestimmten Zeitraum. Dies ist in nachfolgender Gleichung 1a formelmäßig dargestellt:

$$_{ex}^{iZp}v(n)_{alv}^{tid} = \frac{_{ex}^{iZp}v_{CO2}^{mean}}{_{ee}^{iZp}a_{CO2}^{mean}\cdot k} \qquad\qquad\qquad \text{(Gl. 1a)}$$

mit $_{ex}^{iZp}v(n)_{alv}^{tid}$ als der tidalen alveolaren exspiratorischen Atemgasmenge des n-ten Atemhubs, mit $_{ex}^{iZp}v_{CO2}^{mean}$ als der über k Atemhübe gemittelten Menge an $CO_2$ im exspiratorischen Atemgas, und mit $_{ee}^{iZp}a_{CO2}^{mean}$ als dem über k Atemhüben gemittelten Anteil an $CO_2$ im endexspiratorischen Atemgas. Aus der tidalen alveolaren exspiratorischen Atemgasmenge lässt sich bei bekannter tidaler exspiratorischer Atemgasmenge und bekannter tidaler inspiratorischer Atemgasmenge die tidale alveolare inspiratorische Atemgasmenge $_{in}^{iZp}v(n)_{alv}^{tid}$ in an sich bekannter Weise bestimmen. Es gilt sowohl für den inspiratorischen wie für den exspiratorischen Aspekt, dass eine tidale Atemgasmenge die Summe aus tidaler Totraummenge des Atemsystems und tidaler alveolarer Menge ist. Die exspiratorische Tidalmenge an metabolisch-inertem Gas $_{ex}^{iZp}v(n)_{miG}^{tid}$ des n-ten Atemhubs kann dann aus der tidalen alveolaren exspiratorischen Atemgasmenge $_{ex}^{iZp}v(n)_{alv}^{tid}$ und dem Anteil an metabolisch inertem Gas im endexspiratorischen Atemgas $_{ee}^{iZp}a(n)_{miG}^{tid}$ des n-ten Atemhubs gemäß nachfolgender Gleichung 1b ermittelt werden:

$$_{ex}^{iZp}v(n)_{miG}^{tid} = {}_{ex}^{iZp}v(n)_{alv}^{tid} \cdot {}_{ee}^{iZp}a(n)_{miG}^{tid} \qquad\qquad\qquad \text{(Gl. 1b)}$$

Anstelle des tidal berechneten Anteils $_{ee}^{iZp}a(n)_{miG}^{tid}$ kann auch ein über mehrere Atem- hübe gemittelter Anteil an metabolisch-inertem Gas verwendet werden.

Analog hierzu kann die tidale Menge an metabolisch-inertem Gas im inspiratorischen Atemgas $_{in}^{iZp}v(n)_{miG}^{tid}$ auf Grundlage der tidalen alveolaren inspiratorischen Atemgas- menge $_{in}^{iZp}v(n)_{alv}^{tid}$ und dem tidal ermittelten Anteil an metabolisch inertem Gas im inspiratorischen Atemgas $_{in}^{iZp}a(n)_{miG}^{tid}$ des n-ten Atemhubs gemäß Gleichung 1c ermittelt werden:

$$_{in}^{iZp}v(n)_{miG}^{tid} = {}_{in}^{iZp}v(n)_{alv}^{tid} \cdot {}_{in}^{iZp}a(n)_{miG}^{tid} \qquad\qquad\qquad \text{(Gl. 1c)}$$

Wiederum kann anstelle eines tidal ermittelten Anteils ein über mehrere Atemhübe gemittelter Anteilswert verwendet werden. Die tidal ermittelten Anteilswerte sind üblicherweise über einen jeweiligen tidalen Teilatemvorgang: Exspiration und Inspiration, gemittelte Werte.

Endtidale bzw. endexspiratorische Werte werden dabei bevorzugt, da das gegen Ende eines Atemhubs bzw. eines Exspirationsvorgangs beobachtbare endtidale bzw. endexspiratorische Atemgas sicher aus dem stoffwechselnden Bereich der Lunge und sicher nicht aus einem Totraum des Atemsystems stammt.

Es hat sich jedoch herausgestellt, dass beispielsweise für Patienten mit obstruktivpathologischer Lunge die Gleichungen 1a und 1b, wegen deren Fokus auf die endtidale Phase eines Atemhubs, keine optimalen Werte liefern. Daher hat es sich als vorteilhaft herausgestellt, anstelle endexspiratorisch ermittelter Werte Werte zu verwenden, die in einem mittleren Zeitabschnitt der Exspiration ermittelt wurden. Dann geht Gleichung 1a in nachfolgende Gleichung 1d über:

$$\prescript{iZp}{ex}{v}(n)^{tid}_{alv} = \frac{\prescript{iZp}{ex}{v}^{mean}_{CO2}}{\prescript{iZp}{zent}{a}^{mean}_{CO2} \cdot k} \qquad\qquad \text{(Gl. 1d)}$$

wobei $\prescript{iZp}{zent}{a}^{mean}_{CO2}$ ein über k Atemhübe gemittelter Anteilswert von $CO_2$ im exspiratorischen Atemgas ist, deren Einzelwerte jeweils in einem zentralen Zeitbereich der jeweiligen Exspirationsphase bestimmt wurde, also zu einem Zeitpunkt, welcher der zeitlichen Mitte der Exspirationsphase näher liegt als dem Beginn oder dem Ende der Exspirationsphase. Bevorzugt liegt der Erfassungszeitpunkt in einem Zeitbereich, welcher nicht länger als 20 % der Dauer der Exspirationsphase ist und welcher sich symmetrisch um die zeitliche Mitte der Exspirationsphase erstreckt. Besonders bevorzugt ist wenigstens ein Anteilswert, bzw. sind bevorzugt die jeweiligen Anteilswerte in der zeitlichen Mitte der jeweiligen Exspirationsphase ermittelt.

Entsprechendes gilt auch für die Ermittlung des Anteils an metabolisch-inertem Gas im exspiratorischen Atemgas. Gleichung 1b wird dann zur nachfolgenden Gleichung 1e:

$$\prescript{iZp}{ex}{v}(n)^{tid}_{miG} = \prescript{iZp}{ex}{v}(n)^{tid}_{alv} \cdot \prescript{iZp}{zent}{a}(n)^{tid}_{miG} \qquad\qquad \text{(Gl. 1e)}$$

wobei $\prescript{iZp}{zent}{a}(n)^{tid}_{miG}$ ein jeweils in einem zentralen Zeitbereich der jeweiligen Exspirationsphase des n-ten Atemhubs tidal bestimmter Anteil an metabolisch inertem Gas am exspiratorischen Atemgas ist. Es gilt hinsichtlich des bevorzugten Bestimmungszeitpunktes des Anteilswerts das oben zum $CO_2$-Anteilswert Gesagte.

Die Berechnung der tidalen inspiratorischen Menge an metabolisch-inertem Gas kann unverändert nach Gleichung 1c erfolgen.

Erstaunlicherweise kann ein noch genaueres Ergebnis der Tidalmengendifferenz erhalten werden, wenn die einzelnen Tidalmengen der rechten Seite aus Gleichung 1 auf Grundlage einer gemittelten tidalen inspiratorischen Atemgasmenge, multipliziert mit dem mittleren Anteil an metabolisch-inertem Gas der jeweiligen Inspirationsphase, sowie auf Grundlage einer gemittelten tidalen exspiratorischen Atemgasmenge, multipliziert mit dem mittleren Anteil an metabolisch-inertem Gas der jeweiligen Exspirationsphase, erhalten werden.

Eine mittlere tidale inspiratorische Atemgasmenge $\prescript{iZp}{in}{v}^{mean}$ kann dabei erhalten werden durch Mittelwertbildung über mehrere tidale inspiratorische Atemgasmengen hinweg. Da die tidale inspiratorische sowie die tidale exspiratorische Atemgasmenge in der Regel von Änderungen der Atemgaszusammensetzung unbeeinflusst sind, kann die Mittelwertbildung auch über die Grenze zwischen zwei Zufuhrphasen hinweg erfolgen. Die tidale inspiratorische Menge an metabolisch-inertem Gas des n-ten Atemhubs kann dann anstelle von Gleichung 1c gemäß nachfolgender Gleichung 1f ermittelt werden:

$$\prescript{iZp}{in}{v}(n)^{tid}_{miG} = \prescript{iZp}{in}{v}^{mean} \cdot \prescript{iZp}{in}{a}(n)^{tid}_{miG} \qquad\qquad \text{(Gl. 1f)}$$

wobei $\prescript{iZp}{in}{a}(n)^{tid}_{miG}$ wiederum der tidal ermittelte Anteil an metabolisch inertem Gas im inspiratorischen Atemgas des n-ten Atemhubs ist.

Entsprechend kann die tidale exspiratorische Menge an metabolisch-inertem Gas gemäß nachfolgender Gleichung 1g ermittelt werden:

$$\prescript{iZp}{ex}{v}(n)^{tid}_{miG} = \prescript{iZp}{ex}{v}^{mean} \cdot \prescript{iZp}{ex}{a}(n)^{tid}_{miG} \qquad\qquad \text{(Gl. 1g)}$$

wobei ${}_{ex}^{iZp}a(n)_{miG}^{tid}$ wiederum der tidal ermittelte Anteil an metabolisch inertem Gas im exspiratorischen Atemgas des n-ten Atemhubs ist, und wobei ${}_{ex}^{iZp}v^{mean}$ eine durch Mittelwertbildung über mehrere tidale Atemgasmengen hinweg ermittelte mittlere tidale exspiratorische Atemgasmenge ist. Es gilt das oben zur mittleren tidalen inspiratorischen Atemgasmenge Gesagten für die mittlere tidale exspiratorische Atemgasmenge mutatis mutandis entsprechend.

Ein Differenzenwert kann die in einem Atemhub auftretende Tidalmengendifferenz selbst sein. Dann repräsentiert jeder Differenzenwert den Atemhub seiner Tidalmengendifferenz. Um unvermeidliche betragsmäßige Schwankungen der Tidalmengendifferenzen einzelner Atemhübe zu glätten, können die Differenzenwerte Mittelwerte sein, welche Tidalmengendifferenzen einer Mehrzahl von Atemhüben berücksichtigen. Beispielsweise können die Tidalmengendifferenzen gleitende Mittelwerte sein. Zur besseren Vergleichbarkeit berücksichtigt jeder gleitende Mittelwert die gleiche Anzahl von Einzelwerten. Die Mittelwerte können - bevorzugt - arithmetische Mittelwerte umfassen oder sein. Sie können alternativ auch geometrische Mittelwerte umfassen oder sein. Ein über k Atemhübe als gleitender arithmetischer Mittelwert berechneter, den n-ten Atemhub in der i-ten Zufuhrphase repräsentierender Differenzenwert D(n) lässt sich daher formelmäßig darstellen als

$$D(n) = \frac{1}{(k+1)} \cdot \sum_{x=n-k}^{n} {}^{iZp}\Delta v(x)_{miG}^{tid} \qquad \text{(Gl. 2)}$$

wobei ${}^{iZp}\Delta v(x)_{miG}^{tid}$ gemäß Gl. 1 berechnet werden kann.

Da sich die Tidalmengendifferenzen mit fortschreitender Dauer der zweiten Zufuhrphase dem eingangs genannten Offset-Wert annähern, können die Mittelwerte eine Gewichtung aufweisen, vorzugsweise eine Gewichtung, bei welcher die Tidalmengendifferenzen von zeitlich näher am aktuellen Atemhub gelegenen Atemhüben, vorzugsweise einschließlich des aktuellen Atemhubs, stärker gewichtet werden als die tidalen Mengendifferenzen von zeitlich weiter vom aktuellen Atemhub entfernt gelegenen Atemhüben. Bei der Verwendung gleitender Mittelwerte gilt ein Mittelwert als denjenigen Atemhub repräsentierend, für welchen der zeitlich letzte Einzelwert ermittelt wurde, der bei der Berechnung des gleitenden Mittelwerts berücksichtigt wird. Der Vergleich eines Unterschieds von Differenzenwerten mit dem vorbestimmten Differenzen-Schwellenwert kann eine Subtraktion eines ersten Differenzenwerts, welcher einen bestimmten Atemhub repräsentiert, von einem zweiten Differenzenwert umfassen, welcher einen dem vorbestimmten Atemhub unmittelbar nachfolgenden Atemhub repräsentiert. Vorzugsweise ist der unmittelbar nachfolgende Atemhub der jeweils aktuelle Atemhub.

Es sei an dieser Stelle ausdrücklich darauf verwiesen, dass das vorliegend beschriebene Verfahren nicht nur ein zur Beschreibung des Standes der Technik herangezogenes Auswaschverfahren ("Wash-Out") sein muss, sondern auch in Umkehr des Prinzips eines Auswaschverfahrens ein Einspülverfahren ("Wash-In") sein kann. Der zweite Anteil an metabolisch-inertem Gas im zweiten inspiratorischen Atemgas muss daher nicht notwendigerweise niedriger sein als der entsprechende erste Anteil im ersten inspiratorischen Atemgas. Er kann auch höher sein als der erste Anteil. Definiert man die eingangs genannte, während des Ermittlungszeitraums in der zweiten Zufuhrphase auftretende Mengendifferenz als

$${}^{2Zp}\Delta v_{miG} = {}_{in}^{2Zp}v_{miG} - {}_{ex}^{2Zp}v_{miG} \qquad \text{(Gl. 3)}$$

mit ${}^{2Zp}\Delta v_{miG}$ als der während des Ermittlungszeitraums in der zweiten Zufuhrphase auftretenden Mengendifferenz zwischen der Menge an metabolisch-inertem Gas im inspiratorischem Atemgas (diese ist in der vorliegenden Anmeldung auch als Menge an inspiratorischem metabolisch-inertem Gas bezeichnet) und der Menge an metabolisch-inertem Gas im exspiratorischem Atemgas (diese ist in der vorliegenden Anmeldung auch als Menge an exspiratorischem metabolisch-inertem Gas bezeichnet), mit ${}_{in}^{2Zp}v_{miG}$ als der in der zweiten Zufuhrphase verabreichten Menge an inspiratorischem metabolisch-inertem Gas und mit ${}_{ex}^{2Zp}v_{miG}$ als der in der zweiten Zufuhrphase ausgeatmeten Menge an exspiratorischem metabolisch-inertem Gas, dann ist die während der zweiten Zufuhrphase auftretende Mengendifferenz negativ für ein Auswaschverfahren und positiv für ein Einspülverfahren. Denn in der Summe wird beim Auswaschverfahren mehr metabolisch-inertes Gas ausgeatmet als eingeatmet. Beim Einspülverfahren ist es umgekehrt.

Grundsätzlich gilt für die Basisdifferenz das oben zu den Differenzenwerten Gesagte. Die Basisdifferenz kann gebildet sein aus lediglich der Tidalmengendifferenz eines einzelnen Atemhubs, vorzugsweise eines Atemhubs gegen Ende der ersten oder der zweiten Zufuhrphase, besonders bevorzugt der zweiten Zufuhrphase, was jedoch wegen der möglichen Schwankungen der einzelnen tidalen Messwerte zur Bestimmung der Tidalmengendifferenz von Atemhub zu Atemhub nicht bevorzugt ist. Bevorzugt umfasst daher die Basisdifferenz wenigstens einen Mittelwert aus einer Mehrzahl von Tidalmengendifferenzen zwischen jeweils einer tidalen Menge an inspiratorischem metabolisch-inerten Gas und einer tidalen Menge an exspiratorischem metabolisch-inerten Gas für eine Mehrzahl von Atemhüben in der ersten oder/und der zweiten Zufuhrphase. Wiederum kann der angesprochene Mittelwert einen arithmetischen Mittelwert umfassen oder ein solcher sein. Alternativ kann der Mittelwert einen geometrischen Mittelwert umfassen oder ein solcher sein. Dies ermöglicht die Glättung der oben angesprochenen Schwankung von Erfassungswerten. Der Mittelwert kann ein gleitender Mittelwert sein oder kann ein Mittelwert sein, welcher aufgrund einer vorbestimmten Anzahl an Atemhüben nach Verstreichen einer Anzahl von Atemhüben seit dem Beginn der zweiten Zufuhrphase ermittelt wird.

Bevorzugt ist die Basisdifferenz ein Mittelwert aus einer Mehrzahl von Tidalmengendifferenzen von Atemhüben, welche in der zweiten Zufuhrphase erfolgt sind, da sich die Offset-Werte Tidalmengendifferenzen der ersten Zufuhrphase und der zweiten Zufuhrphase betragsmäßig unterscheiden können und sich in der Praxis tatsächlich in der Regel unterscheiden. Dann repräsentiert die Basisdifferenz den Offset-Wert der zweiten Zufuhrphase, was eine höhere Genauigkeit der ermittelten FRC verspricht als eine Basisdifferenz, die den Offset-Wert der ersten Zufuhrphase repräsentiert. Da jedoch in der Regel die Offset-Werte der ersten und der zweiten Zufuhrphase das gleiche Vorzeichen haben, liefert die Verwendung einer Basisdifferenz auf Grundlage von Atemhüben der ersten Zufuhrphase, die folglich den Offset-Wert der ersten Zufuhrphase repräsentiert, immer noch eine bessere Genauigkeit als keine Berücksichtigung einer Basisdifferenz.

Eine als arithmetischer Mittelwert für die i-te Zufuhrphase berechnete Basisdifferenz $^{iZp}B$ lässt sich daher in Anlehnung an die Gleichung 2 für den Differenzenwert schreiben als:

$$^{iZp}B = \frac{1}{(m+1)} \cdot \sum_{x=n_0-m}^{n_0} {}^{iZp}\Delta v(x)_{miG}^{tid} \qquad \text{(Gl. 4)}$$

wobei $n_0$ besonders bevorzugt die Nummer des letzten Atemhubs des Ermittlungszeitraums oder der i-ten Zufuhrphase (Zp) ist, und wobei $m$ die Anzahl an Einzelwerten von Tidalmengendifferenzen ist, die zur Mittelwertbildung der Basisdifferenz berücksichtigt werden. Allgemein gilt bevorzugt, dass der Betrag des Abstandes zwischen $n_0$ und der Nummer des letzten Atemhubs des Ermittlungszeitraums oder der i-ten Zufuhrphase kleiner ist als $n_0 - m - 1$. Letzteres ist der Abstand der Nummer $n_0 - m$ des ersten Atemhubs, welcher bei der Bildung der Basisdifferenz berücksichtigt wird, vom ersten Atemhub des Ermittlungszeitraums oder der i-ten Zufuhrphase. Bevorzugt gilt, dass der erste Atemhub des Ermittlungszeitraums mit dem ersten Atemhub der zweiten Zufuhrphase identisch ist.

Da sich der gewünschte Gleichgewichtszustand, in welchem sich Tidalmengendifferenzen zwischen jeweils einer tidalen Menge an inspiratorischem metabolisch-inerten Gas und einer tidalen Menge an exspiratorischem metabolisch-inerten Gas betragsmäßig über mehrere Atemzüge hinweg möglichst wenig ändern, mit zunehmenden Abstand vom Beginn einer Zufuhrphase einstellt, ist es zur Erzielung einer möglichst hohen Genauigkeit in der Ermittlung einer FRC bevorzugt, dass die Basisdifferenz einen Mittelwert aus einer Mehrzahl von Tidalmengendifferenzen zwischen jeweils einer tidalen Menge an inspiratorischem metabolisch-inerten Gas und einer tidalen Menge an exspiratorischem metabolisch-inerten Gas für eine Mehrzahl von Atemhüben in einem in der ersten Zufuhrphase gelegenen zeitlichen Anfangs-Erfassungsabschnitt umfasst, wobei der Anfangs-Erfassungsabschnitt dem Anfang der zweiten Zufuhrphase näher gelegen ist als dem Anfang ersten Zufuhrphase. Dies gilt also für den Fall, dass die Basisdifferenz auf Erfassungswerten von Atemhüben der ersten Zufuhrphase beruht.

Zusätzlich oder alternativ kann aus demselben Grunde eines möglichst genauen Ermittlungsergebnisses der FRC vorgesehen sein, dass die Basisdifferenz einen Mittelwert aus einer Mehrzahl von Tidalmengendifferenzen zwischen jeweils einer tidalen Menge an inspiratorischem metabolisch-inerten Gas und einer tidalen Menge an exspiratorischem metabolisch-inerten Gas für eine Mehrzahl von Atemhüben in einem in der zweiten Zufuhrphase gelegenen zeitlichen End-Erfassungsabschnitt umfasst, wobei der End-Erfassungsabschnitt dem Ende der zweiten Zufuhrphase näher gelegen ist als deren Anfang. Da der Ermittlungszeitraum in der zweiten Zufuhrphase gelegen ist, ist die Ermittlung der Basisdifferenz aus Tidalmengendifferenzen von Atemhüben der zweiten Zufuhrphase aus den oben bereits geschilderten Gründen einer erzielbaren höheren Genauigkeit bei der Ermittlung der FRC bevorzugt.

Alternativ oder zusätzlich kann die Ermittlung der Basisdifferenz erst ab einem betragsmäßigen Unterschreiten eines Basis-Schwellenwerts durch eine Tidalmengendifferenz zwischen einer tidalen Menge an inspiratorischem metabolisch-inertem Gas und einer tidalen Menge an exspiratorischem metabolisch-inertem Gas erfolgen. Dadurch ist sichergestellt, dass die Basisdifferenz nur auf Grundlage von Atemhüben mit Tidalmengendifferenzen erfolgt, bei denen die Patientenlunge größtenteils ausgewaschen bzw. eingespült ist, je nach angewendetem Verfahren, sich mithin der Gasaustausch so nahe an dem oben genannten Gleichgewichtszustand befindet, dass eine bezüglich des sich mit der verwendeten Beatmungsvorrichtung oder/und in der jeweiligen Beatmungssituation einstellenden Offset-Werts aussagekräftige Basisdifferenz ermittelbar ist. Wie nahe sich die Patientenlunge bei Beginn einer Ermittlung der Basisdifferenz an dem Gleichgewichtszustand befindet, wird durch Wahl des Betrags des Basis-Schwellenwerts festgelegt. In der Regel wird bei sinnvoller Wahl des Basis-Schwellenwerts auch die obige Bedingung erfüllt sein, dass die Atemhübe, auf deren Erfassungswerten die Ermittlung der Basisdifferenz beruht, mehrheitlich oder bevorzugt vollständig näher am Ende der Zufuhrphase gelegen sind, in welcher sich die Atemhübe befinden, als an deren Anfang.

Die Genauigkeit der Ermittlung einer FRC eines Patienten lässt sich jedoch noch weiter erhöhen, indem die Basisdifferenz detaillierter bzw. differenzierter betrachtet und berechnet wird.

Ohne auf die physiologisch-physikalischen Zusammenhänge im Einzelnen einzugehen, hat der Offset-Wert in Versuchen wiederholbar eine Abhängigkeit von dem Anteil an metabolisch-inertem Gas im Atemgas gezeigt. Daher kann die mit dem vorliegend vorgeschlagenen Verfahren ermittelte FRC dann noch näher an der zur Verifizierung mit bekannten anerkannten Ermittlungsverfahren ermittelten FRC liegen, wenn die Basisdifferenz zumindest zeitabschnittsweise abhängig vom Anteil an metabolisch-inertem Gas im Atemgas ermittelt und zur Ermittlung der FRC verwendet wird.

Dies kann in einer wegen der dabei erzielten Genauigkeit der FRC-Ermittlung bevorzugten Ausgestaltung des vorliegenden FRC-Ermittlungsverfahrens dadurch erfolgen, dass wenigstens für eine Mehrzahl von Atemhüben eine tidale Basisdifferenz ermittelt wird, welche eine Funktion des Anteils an metabolisch-inertem Gas im Atemgas während des jeweiligen gesamten Atemhubs ist. Bevorzugt wird die tidale Basisdifferenz als Funktion eines über den jeweiligen Atemhub gemittelten tidalen Anteils an metabolisch-inertem Gas im Atemgas ermittelt, wobei der gemittelte tidale Anteil bevorzugt sowohl den Anteil im inspiratorischen, wie auch im exspiratorischen Atemgas berücksichtigt, um den Auswasch- bzw. den Einspülvorgang abzubilden.

Genauer kann die tidale Basisdifferenz ein Verhältnis einer Funktion eines über den jeweiligen gesamten Atemhub gemittelten tidalen Anteils an metabolisch-inertem Gas im Atemgas zu der eingangs genannten Anteilsdifferenz sein. Die Funktion kann dabei beispielsweise der über den jeweiligen Atemhub gemittelte Anteil an metabolisch-inertem Gas im Atemgas selbst sein oder, bevorzugt, kann die Differenz zwischen dem über den jeweiligen Atemhub gemittelten Anteil an metabolisch-inertem Gas im Atemgas und der ersten Anteilsgröße sein.

Bevorzugt weist die tidale Basisdifferenz während der zweiten Zufuhrphase Werte auf, welche eine gemittelte, vorzugsweise gemäß obiger Gleichung 4, Basisdifferenz $^{2Zp}B$ für die zweite Zufuhrphase betragsmäßig nicht übersteigen. Da vor der zweiten Zufuhrphase, also in der ersten Zufuhrphase, eine gemittelte, vorzugsweise wiederum gemäß obiger Gleichung 4, Basisdifferenz $^{1Zp}B$ für die erste Zufuhrphase einschlägig ist, nimmt die tidale Basisdifferenz während der zweiten Zufuhrphase besonders bevorzugt nur Werte an, welche betragsmäßig zwischen $^{1Zp}B$ und $^{2Zp}B$ liegen, die jeweiligen Grenzen eingeschlossen.

Üblicherweise reichen zur Ermittlung der FRC tidale Werte nur der zweiten Zufuhrphase aus. Folglich reicht es aus, eine tidale Basisdifferenz $^{2Zp}B^{tid}(n)$ nur für Atemhübe der zweiten Zufuhrphase zu berechnen.

Mit $^{1Zp}_{in}A_{miG}$ als erster Anteilsgröße und mit $^{2Zp}_{in}A_{miG}$ als zweiter Anteilsgröße kann man die tidale Basisdifferenz des n-ten Atemhubs der zweiten Zufuhrphase formelmäßig allgemein angeben als:

$$^{2Zp}B^{tid}(n) \sim \frac{f\left(^{2Zp}a(n)^{tid}_{miG}\right)}{^{2Zp}_{in}A_{miG} - {}^{1Zp}_{in}A_{miG}} \qquad \text{(Gl. 5)}$$

mit f als Funktion und mit $^{2Zp}a(n)^{tid}_{miG}$ als dem über den n-ten Atemhub der zweiten Zufuhrphase gemittelten tidalen Anteil des metabolisch-inerten Gas am gesamten tidalen Atemgas. Der tidale Anteil wird daher bevorzugt über die inspiratorische und über die exspiratorische Phase hinweg gemittelt. Die oben als besonders bevorzugt genannte Methode zur Berechnung der tidalen Basisdifferenz des n-ten Atemhubs in der zweiten Zufuhrphase ist formelmäßig in der nachfolgenden Gleichung 6 dargestellt:

$$^{2Zp}B^{tid}(n) = {}^{1Zp}B + \left(^{2Zp}B - {}^{1Zp}B\right)\left(\frac{^{2Zp}a(n)^{tid}_{miG} - {}^{1Zp}_{in}A_{miG}}{^{2Zp}_{in}A_{miG} - {}^{1Zp}_{in}A_{miG}}\right) \qquad \text{(Gl. 6)}$$

wobei bevorzugt $^{1Zp}B$ und $^{2Zp}B$ bevorzugt gemäß obiger Gleichung 4 berechnet werden und wobei $^{2Zp}_{in}A - {}^{1Zp}_{in}A$ im Nenner $-\Delta A$ aus unten stehender Gleichung 9 entspricht und daher bevorzugt unter Verwendung der unten näher erläuterten Gleichung 9 als $(-1 \cdot \Delta A)$ berechnet wird.

Bevorzugt wird die korrigierte Mengendifferenz aus einer Summe von korrigierten Tidalmengendifferenzen gebildet. Bei der bevorzugten Ausführung des vorliegenden Verfahrens durch eine Beatmungsvorrichtung während eines Beatmungsbetriebs werden ohnehin mit jedem Atemhub Werte des Atemgases erfasst, insbesondere sowohl des inspiratorischen wie auch des exspiratorischen Atemgases. Daher ist es von Vorteil, die ohnehin vorhandenen tidalen, also auf den einzelnen Atemhub bezogenen Erfassungswerte auch zur Ermittlung der FRC zu nutzen. Dann kann die korrigierte Mengendifferenz einer Summe von korrigierten Tidalmengendifferenzen über eine Anzahl an Atemhüben im Ermittlungszeitraum entsprechen, wobei für jeden Atemhub eine korrigierte Tidalmengendifferenz zwischen einer tidalen Menge an inspiratorischem metabolisch-inertem Gas und einer tidalen Menge an exspiratorischem metabolisch-inertem Gas gebildet wird aus einer Differenz einer Tidalmengendifferenz dieses Atemhubs und einer dem Atemhub zugeordneten Basisdifferenz, wobei für jeden Atemhub die Tidalmengendifferenz gebildet wird durch die Differenz zwischen einer tidalen Menge an inspiratorischem metabolisch-inerten Gas und einer tidalen Menge an exspiratorischem metabolisch-inerten Gas dieses Atemhubs. Die korrigierte Mengendifferenz, lässt sich daher in einer bevorzugten Ausführungsform des Verfahrens formelmäßig wie folgt darstellen:

$$_{korr}^{2Zp}\Delta v_{miG} = \sum_{x=c_0}^{n_0} \left( {}^{2Zp}\Delta v(x)_{miG}^{tid} - {}^{iZp}B \right) \qquad\qquad \text{(Gl. 7a)}$$

wobei $_{korr}^{2Zp}\Delta v_{miG}$ die korrigierte Mengendifferenz ist und ${}^{2Zp}\Delta v(x)_{miG}^{tid}$ die Tidalmengendifferenz des x-ten Atemhubs in dem vollständig in der zweiten Zufuhrphase gelegenen Ermittlungszeitraum ist. $n_0$ ist die Nummer des letzten Atemhubs des Ermittlungszeitraums, $c_0$ ist die Nummer des ersten Atemhubs des Ermittlungszeitraums innerhalb der zweiten Zufuhrphase. Wenn, was bevorzugt ist, $c_0 = 1$ gewählt wird, ist der erste Atemhub des Ermittlungszeitraums auch der erste Atemhub der zweiten Zufuhrphase. Als Basisdifferenz ${}^{iZp}B$ wird bevorzugt eine gemäß obiger Gl. 4 aus Atemhüben der zweiten Zufuhrphase ermittelte Basisdifferenz ${}^{2Zp}B$ herangezogen. Es kann jedoch, wie oben erläutert, auch eine gemäß Gl. 4 aus Atemhüben der ersten Zufuhrphase ermittelte Basisdifferenz ${}^{1Zp}B$ herangezogen werden, was jedoch wegen der geringeren Genauigkeit der hieraus ermittelbaren FRC weniger bevorzugt ist.

Wegen der besonders hohen Genauigkeit in der FRC-Ermittlung wird die korrigierte Mengendifferenz bevorzugt mit folgender modifizierter Gleichung 7b berechnet:

$$_{korr}^{2Zp}\Delta v_{miG} = \sum_{x=c_0}^{n_0} \left( {}^{2Zp}\Delta v(x)_{miG}^{tid} - {}^{2Zp}B^{tid}(x) \right) \qquad\qquad \text{(Gl. 7b)}$$

wobei ${}^{2Zp}B^{tid}(x)$ bevorzugt gemäß Gleichung 6 ermittelt wird.

Auch der erste oder/und der zweite Anteil an metabolisch-inertem Gas im ersten bzw. im zweiten inspiratorischen Atemgas kann bzw. können während der ersten bzw. während der zweiten Zufuhrdauer betragsmäßig schwanken. Grundsätzlich kann als erste Anteilsgröße ein zu einem bestimmten Zeitpunkt erfasster erster Anteil als einziger Erfassungswert verwendet werden. Gleiches gilt entsprechend für die zweite Anteilsgröße. Um jedoch eventuell auftretende betragsmäßige Schwankungen im ersten oder/und im zweiten Anteil glätten zu können, ist es bevorzugt, wenn die erste Anteilsgröße einen über eine Mehrzahl von Atemhüben in der ersten Zufuhrphase hinweg gebildeten Mittelwert des ersten Anteils an dem metabolisch-inerten Gas im ersten inspiratorischen Arbeitsgas umfasst oder ist.

Zusätzlich oder alternativ kann aus demselben Grunde die zweite Anteilsgröße einen über eine Mehrzahl von Atemhüben in der zweiten Zufuhrphase hinweg gebildeten Mittelwert des zweiten Anteils an dem metabolisch-inerten Gas im zweiten inspiratorischen Arbeitsgas umfassen oder sein.

Wiederum gilt, dass der Mittelwert einen arithmetischen Mittelwert umfassen oder ein solcher sein kann. Ebenso kann der Mittelwert einen geometrischen Mittelwert umfassen oder ein solcher sein. Der Mittelwert kann gewichtet sein.

Da idealerweise sich der erste Anteil an metabolisch-inertem Gas am ersten inspiratorischen Atemgas während der ersten Zufuhrphase nicht ändert, und da idealerweise gleiches für den zweiten Anteil während der zweiten Zufuhrphase gilt, spielt es in erster Näherung eine untergeordnete Rolle, auf welchen Atemhüben der ersten bzw. der zweiten Zufuhrphase die erste und die zweite Anteilsgröße bestimmt werden. Treten jedoch betragsmäßige Schwankungen im ersten oder/und im zweiten Anteil während der ersten bzw. der zweiten Zufuhrphase auf, kann das Ermittlungsergebnis der FRC trotz dieser Schwankungen dadurch mit hoher Genauigkeit erhalten werden, dass die Mehrzahl von Atemhüben, über welche hinweg die erste Anteilsgröße als Mittelwert ermittelt wird, näher am Anfang der zweiten Zufuhrphase gelegen ist als am Anfang der ersten Zufuhrphase. Denn je näher zeitlich das erste inspiratorische Atemgas zum Beginn der zweiten Zufuhrphase betrachtet wird, desto größer ist der Einfluss des betrachteten Atemgases auf das Auswaschen bzw. Einspülen von metabolisch-inertem Gas während der zweiten Zufuhrphase.

Zusätzlich oder alternativ ist zur Erhöhung der Genauigkeit des Ermittlungsergebnisses der FRC von Vorteil, wenn die Mehrzahl von Atemhüben, über welche hinweg die zweite Anteilsgröße als Mittelwert ermittelt wird, näher am Ende des Ermittlungszeitraums oder der zweiten Zufuhrphase gelegen ist als an deren Anfang. Dies liegt daran, dass der zweite Anteil an metabolisch-inertem Gas im zweiten inspiratorischen Atemgas einen umso stärkeren Einfluss auf den eingangs beschriebenen Offset-Wert in der zweiten Zufuhrphase hat, je näher er am Ende des Ermittlungszeitraums gelegen ist. Ist der Ermittlungszeitraum beendet, haben nachfolgende Atemhübe keinen Einfluss mehr auf die Ermittlung der FRC. Dennoch kann zur Vereinfachung der Steuerung anstelle des Endes des Ermittlungszeitraums das Ende der zweiten Zufuhrphase gewählt werden, sodass die zweite Anteilsgröße unabhängig vom Ende des Ermittlungszeitraums bestimmt werden kann.

Eine bevorzugte als arithmetischer Mittelwert berechnete Anteilsgröße kann formelmäßig wie folgt dargestellt werden:

$$\prescript{iZp}{in}{A}_{miG} = \frac{1}{(q+1)} \cdot \sum_{x=p-q}^{p} \prescript{iZp}{in}{a}(x)_{miG}^{tid} \qquad \text{(Gl. 8)}$$

wobei $\prescript{iZp}{}{A}_{miG}$ für i = 1 die erste Anteilsgröße und für i = 2 die zweite Anteilsgröße ist, wobei $\prescript{iZp}{}{a}(x)_{miG}^{tid}$ der Anteil an metabolisch-inertem Gas im inspiratorischen Atemgas des x-ten Atemhubs in der i-ten Zufuhrphase ist und wobei p und q positive ganzzahlige Konstanten mit p > q sind, welche die Anzahl an Werten angeben, jeweils während eines anderen Atemhubs erfasst, über die die i-te Anteilsgröße durch Mittelwertbildung errechnet wird. Bevorzugt sind p und q als Nummern eines Atemhubs derart gewählt, dass für die erste Zufuhrphase gilt, dass der Abstand zwischen dem letzten Atemhub und dem p-ten Atemhub der ersten Zufuhrphase größer ist als der Abstand des q-ten Atemhubs vom ersten Atemhub der ersten Zufuhrphase.

Die Gleichung 8 ist für die Berechnung des Anteils an metabolisch-inertem Gas im inspiratorischen Atemgas angegeben. Mit Gleichung 8 lassen sich grundsätzlich alle Anteile einzelner Gasbestandteile einer Gasmischung ausrechnen, und zwar sowohl im inspiratorischen wie auch im exspiratorischen Atemgas.

Da sich mit zunehmendem zeitlichen Abstand vom Beginn der zweiten Zufuhrphase die Werte der Anteile an metabolisch-inertem Gas im inspiratorischen und im exspiratorischen Atemgas angleichen, - denn mit zunehmendem Abstand vom Beginn der zweiten Zufuhrphase ist zuvor eingespültes metabolisch-inertes Gas ausgewaschen oder zuvor ausgewaschenes metabolisch-inertes Gas eingespült - repräsentiert auch der mit ausreichend großem Abstand vom Beginn der zweiten Zufuhrphase ermittelte Anteil an metabolisch-inertem Gas im exspiratorischen Atemgas den Anteil an metabolisch-inertem Gas im inspiratorischen Atemgas und kann daher als zweite Anteilsgröße herangezogen werden. Gleiches gilt für die Verwendung eines mit ausreichendem Abstand vom Beginn der ersten Zufuhrphase ermittelten Anteils des metabolisch-inerten Gases am exspiratorischen Atemgas zur Bestimmung der ersten Anteilsgröße.

Für die zweite Zufuhrphase sind p und q bevorzugt derart gewählt, dass der Abstand zwischen dem letzten Atemhub und dem p-ten Atemhub der zweiten Zufuhrphase oder des Ermittlungszeitraums größer ist als der Abstand des q-ten Atemhubs der zweiten Zufuhrphase vom ersten Atemhub der zweiten Zufuhrphase oder des Ermittlungszeitraums.

Für derart gewählte Werte von p und q gilt Gleichung 8 als modifizierte Gleichung 8a auch für die Summation von Anteilen metabolisch-inerten Gases im exspiratorischen Atemgas oder im Atemgas insgesamt:

$$\prescript{iZp}{in}{A}_{miG} = \frac{1}{(q+1)} \cdot \sum_{x=p-q}^{p} \prescript{iZp}{ex}{a}(x)_{miG}^{tid} \approx \frac{1}{(q+1)} \cdot \sum_{x=p-q}^{p} \prescript{iZp}{}{a}(x)_{miG}^{tid} \quad \text{(Gl. 8a)}$$

Analog zum ersten inspiratorischen Atemgas ist in der vorliegenden Anmeldung während der ersten Zufuhrphase ausgeatmetes Atemgas auch als erstes exspiratorisches Atemgas und während der zweiten Zufuhrphase ausgeatmetes Atemgas auch als zweites exspiratorisches Atemgas bezeichnet. Der Einfachheit halber sei nachfolgend nur Gleichung 8 weiter verwendet, wenngleich auch Gleichung 8a verwendbar wäre.

Mit Gleichung 8 lässt sich die eingangs erwähnte Anteilsdifferenz $\Delta A_{miG}$ in einer bevorzugten Ausführungsform des Verfahrens formelmäßig wie folgt notieren:

$$\Delta A_{miG} = {}^{1Zp}_{in}A_{miG} - {}^{2Zp}_{in}A_{miG}$$

$$\equiv \left( \frac{1}{({}^{1Zp}q + 1)} \cdot \sum_{x={}^{1Zp}p-{}^{1Zp}q}^{{}^{1Zp}p} {}^{1Zp}_{in}a(x)^{tid}_{miG} \right)$$

$$- \left( \frac{1}{({}^{2Zp}q + 1)} \cdot \sum_{x={}^{2Zp}p-{}^{2Zp}q}^{{}^{2Zp}p} {}^{2Zp}_{in}a(x)^{tid}_{miG} \right)$$

(Gl. 9)

Dabei können ${}^{1Zp}p$ und ${}^{2Zp}p$ sich betragsmäßig ebenso unterscheiden wie ${}^{1Zp}q$ und ${}^{2Zp}q$. Die jeweiligen Werte von p oder/und von q können für beide Zufuhrphasen auch die gleichen sein.

Das Ermitteln der funktionalen Restkapazität erfolgt bevorzugt auf Grundlage eines Quotienten aus der korrigierten Mengendifferenz und der Anteilsdifferenz. Die funktionale Restkapazität FRC einer Patientenlunge lässt sich in obiger Notation formelmäßig wie folgt darstellen:

$$FRC = \frac{{}^{2Zp}_{korr}\Delta v_{miG}}{\Delta A_{miG}}$$

(Gl. 10)

Gemäß einer bevorzugten Ausführungsform des Verfahrens ist die funktionale Restkapazität FRC einer Patientenlunge mit Gleichungen 10, 7a und 9:

$$FRC = \frac{\sum_{x=c_0}^{n_0} {}^{2Zp}\Delta v(x)^{tid}_{miG} - {}^{iZp}B}{{}^{1Zp}A_{miG} - {}^{2Zp}A_{miG}}$$

(Gl. 11a)

oder, wegen der höheren Genauigkeit bevorzugt mit Gleichungen 10, 7b und 9:

$$FRC = \frac{\sum_{x=c_0}^{n_0} \left( {}^{2Zp}\Delta v(x)^{tid}_{miG} - {}^{2Zp}B^{tid}(x) \right)}{{}^{1Zp}A_{miG} - {}^{2Zp}A_{miG}}$$

(Gl. 11b)

wobei $\Delta A$ im Nenner gemäß Gleichung 9 ermittelt wird.

Bevorzugt wird das vorliegende Verfahren zur Ermittlung der FRC während einer künstlichen Beatmung eines Patienten ausgeführt, sodass die Atemhübe, während welchen die obigen Werte zur Berechnung der FRC erfasst werden, Atemhübe zur künstlichen Beatmung des Patienten sind. Die künstliche Beatmung des Patienten dauert üblicherweise nach Abschluss des Ermittlungszeitraums oder nach Ende der zweiten Zufuhrphase fort. Bevorzugt erfolgte künstliche Beatmung bereits über mehrere Atemhübe hinweg, bevor das vorliegend diskutierte Verfahren zur Ermittlung einer FRC beginnt. Zur Sicherstellung der Gasaustauschfähigkeit der Patientenlunge ist es daher bevorzugt, wenn am Ende einer Exspirationsphase einer Mehrzahl von Atemhüben der zweiten Zufuhrphase ein Atemwegsdruck im Atemweg des Patienten oder/und in einem proximalen Bereich einer Beatmungsleitung der PEEP ist. Der PEEP ist ein Überdruck im Verhältnis zum Umgebungsdruck, welcher verhindert, dass Alveolen der Patientenlunge am Ende einer Exspirationsphase kollabieren.

Während im Stand der Technik häufig lediglich messtechnisch leichter zu erfassende Werte am exspiratorischen Atemgas erfasst werden und die ebenfalls benötigten Werte des inspiratorischen Atemgases aus den erfassten exspiratorischen Atemgaswerten abgeleitet werden, ermöglicht die Verwendung einer Beatmungsvorrichtung, wie sie oben bereits genannt ist und wie sie unten weiter detailliert angegeben ist, zur Durchführung des Verfahrens zur FRC-Ermittlung die sensorische Erfassung sowohl des inspiratorischen Atemgasstroms als auch des exspiratorischen Atemgasstroms. Daher umfasst das Verfahren bevorzugt das sensorische Erfassen sowohl des inspiratorischen Atemgasstroms als auch des exspiratorischen Atemgasstroms. Dies hat den Vorteil, dass die in der Fachwelt üblicherweise zur Ableitung von inspiratorischen Atemgaswerten aus gemessenen exspiratorischen Atemgaswerten herangezogene Haldane-Transformation nicht verwendet werden muss. Folglich kommt es nicht darauf an, ob die der Haldane-Transformation zugrunde liegenden vereinfachenden Annahmen tatsächlich erfüllt sind oder nicht. Selbst bei Auftreten der üblichen Störungseinflüsse, wie Feuchtigkeit, Feuchtigkeitsniederschlag an Sensoren in einer Beatmungsleitungsanordnung der Beatmungsvorrichtung kann daher das sensorische Erfassen sowohl des inspiratorischen als auch des exspiratorischen Atemgasstroms einen Genauigkeitsvorteil in der Ermittlung der FRC gegenüber der Verwendung der Haldane-Transformation bieten. Zum einen können die Voraussetzungen der Haldane-Transformation nicht vorliegen. Zusätzlich oder alternativ können zum anderen die oben genannten Störungseinflüsse auch auf die sensorische Erfassung nur des exspiratorischen Atemgases einwirken.

Grundsätzlich kann das metabolisch-inerte Gas unmittelbar von einem Sensor erfasst werden, um dessen Anteil am inspiratorischen oder/und am exspiratorischen Atemgas zu ermitteln. Das metabolisch-inerte Gas kann ein Edelgas sein, wie beispielsweise Helium (He), oder kann ein vom lebenden Organismus nicht oder nahezu nicht verstoffwechseltes Gas sein, wie beispielsweise der in der Luft ohnehin vorhandene Stickstoff ($N_2$), oder kann Schwefelhexafluorid ($SF_6$) sein. Bevorzugt ist das metabolisch-inerte Gas Stickstoff, da dann Umgebungsluft als Basisgas für inspiratorisches Atemgas verwendet werden kann. Zur Veränderung des Anteils an Stickstoff einem auf Umgebungsluft basierenden inspiratorischen Atemgas kann dem inspiratorischen Atemgas reiner Sauerstoff ($O_2$) oder Gas mit einem höheren Sauerstoffanteil als jenem von Luft beigemischt werden. So hat bevorzugt ein Gas aus dem ersten oder dem zweiten inspiratorischen Atemgas den natürlichen Stickstoffgehalt der Umgebungsluft und das jeweils andere inspiratorische Atemgas hat einen niedrigeren Stickstoffgehalt. Dadurch ist sichergestellt, dass der Patient sowohl das erste als auch das zweite inspiratorische Atemgas verträgt. Es soll jedoch nicht ausgeschlossen sein, dass der Stickstoffgehalt sowohl im ersten als auch im zweiten inspiratorischen Atemgas niedriger als in der Umgebungsluft ist, insbesondere wenn eine Erkrankung eines künstlich beatmeten Patienten einen höheren Sauerstoffgehalt im inspiratorischen Atemgas erfordert.

Insbesondere dann, wenn Stickstoff als natürlicher Bestandteil der Umgebungsluft als das metabolisch-inerte Gas verwendet wird, kann der Stickstoffanteil im Atemgas, sei es nun inspiratorisch oder exspiratorisch, mittelbar mit ausreichender Genauigkeit erfasst werden, indem unmittelbar der Sauerstoffgehalt und der Kohlendioxidgehalt des jeweilige Atemgases sensorisch erfasst werden. Da mit guter Näherung davon ausgegangen werden kann, dass ein Atemgas, insbesondere ein auf Umgebungsluft basierendes Atemgas, nahezu nur aus Stickstoff, Sauerstoff und Kohlendioxid besteht, lässt sich der Stickstoffgehalt des sensorisch erfassten Atemgases als der Restgehalt des Atemgases bestimmen, der nicht Sauerstoffgehalt oder Kohlendioxidgehalt ist. Formelmäßig ausgedrückt bedeutet dies für den Stickstoffgehalt $^{iZp}a(x)^{tid}_{N_2}$ in Prozent, im x-ten Atemhub der i-ten Zufuhrphase:

$$^{iZp}a(x)^{tid}_{N_2} = 1 - {}^{iZp}a(x)^{tid}_{O_2} - {}^{iZp}a(x)^{tid}_{CO_2} \qquad \text{(Gl. 12)}$$

wobei $^{iZp}a(x)^{tid}_{O_2}$ der Sauerstoffgehalt des Atemgases und $^{iZp}a(x)^{tid}_{CO_2}$ der Kohlendioxidgehalt des Atemgases im selben Atemhub sind.

Die bezeichneten Anteile in Prozent können Volumenanteile, Massenanteile oder Molanteile sein, je nachdem welcher Anteil sensorisch erfasst wird. Bevorzugt sind es Volumenanteile in Volumenprozent. Ebenso können die in der vorliegenden Anmeldung bezeichneten Mengen Volumina, Massen oder Molmengen sein. Bevorzugt sind die Mengen Volumina.

Wie bereits mehrfach erläutert wurde, wird das vorliegend vorgestellte Verfahren bevorzugt von einer Beatmungsvorrichtung während einer künstlichen Beatmung eines Patienten durchgeführt.

Die erste Atemgasquelle ist bevorzugt eine zur Umgebung hin offene Ansaugöffnung, durch welche Umgebungsluft angesaugt werden kann. Die erste Atemgasquelle kann jedoch auch ein Vorratsbehälter zur Aufnahme von erstem inspiratorischem Atemgas sein oder kann eine Anschlusskupplung zur Verbindung mit einer klinischen Hausinstallation zur Versorgung mit erstem inspiratorischem Atemgas sein. In zahlreichen Kliniken sind im Klinikgebäude Versorgungsleitungen mit definierten Anschlussgegenkupplungen fest installiert, wobei die Anschlussgegenkupplungen für die Anschlusskupplung der Beatmungsvorrichtung zur Herstellung einer das erste inspiratorische Atemgas leitenden Verbindung zugänglich bereitstehen.

Bevorzugt ist die zweite Atemgasquelle mit Verweis auf die obigen Ausführungen zum Verfahren ein Sauerstoffbehälter, aus welchem Sauerstoff in das Atemgas der ersten Atemgasquelle zugeführt werden kann. Alternativ oder zusätzlich kann auch die zweite Atemgasquelle eine Anschlusskupplung zur Verbindung mit einer klinischen Hausinstallation sein. Das Maß an Zuführung von Gas der zweiten Atemgasquelle in Gas der ersten Atemgasquelle ist an der Mischvorrichtung einstellbar. Es kann in einer einfachen Ausführungsform ausreichen, die Mischvorrichtung zwischen einem Sperrzustand und einem definierten Öffnungszustand zu schalten, wobei im Sperrzustand kein Gas aus der zweiten Atemgasquelle in Gas aus der ersten Atemgasquelle strömen kann, und wobei im definierten Öffnungszustand

14

pro Zeiteinheit eine stets gleiche Menge an Gas von der zweiten Atemgasquelle in Gas der ersten Atemgasquelle einströmt. Bevorzugt ist die Mischvorrichtung jedoch wenigstens stufenweise von dem Sperrzustand in unterschiedliche definierte Öffnungszustände oder besonders bevorzugt stufenlos in unterschiedliche Öffnungszustände bringbar, sodass wenigstens ein inspiratorisches Atemgas aus dem ersten und dem zweiten inspiratorischen Atemgas, vorzugsweise beide inspiratorischen Atemgase, stufenlos an die Bedürfnisse des jeweils beatmeten Patienten anpassbar sind.

Die Druckveränderungsvorrichtung kann ein das Atemgas, wenigstens oder nur das inspiratorische Atemgas, in der Beatmungsleitungsanordnung förderndes Gebläse sein. Dies gilt insbesondere dann, wenn Umgebungsluft als erstes oder zweites inspiratorisches Atemgas oder als Atemgaskomponente verwendet wird.

Die Druckveränderungsvorrichtung kann jedoch zusätzlich oder alternativ ein Druck minderndes Ventil umfassen, dessen Zustand und damit dessen Druck mindernde Wirkung durch die Steuervorrichtung veränderbar ist.

Die Beatmungsleitungsanordnung dient dazu, inspiratorisches Atemgas von der ersten oder/und der zweiten Atemgasquelle zum Patienten und exspiratorisches Atemgas vom Patienten weg zu leiten. Der Atemgasauslass und der Atemgaseinlass können ein und dieselbe Formation sein, beispielsweise die proximale Öffnung eines Endotrachealtubus. Sie können jedoch auch verschiedene Öffnungen sein.

Die Beatmungsleitungsanordnung kann körperlich gesondert ausgebildete Abschnitte für inspiratorisches und für exspiratorisches Atemgas aufweisen. Die Abschnitte können durch einen sogenannten Y-Verbinder zu einem gemeinsamen sowohl für exspiratorisches als auch für inspiratorisches Atemgas genutzten Leitungsabschnitt zusammengeführt sein. Der sowohl für exspiratorisches als auch für inspiratorisches Atemgas genutzte Leitungsabschnitt ist bevorzugt ein Leitungsabschnitt, welcher zwischen dem Y-Verbinder und dem Patienten angeordnet ist, also insbesondere bis zum Atemgaseinlass und zum Atemgasauslass reicht.

Die Flusssensoranordnung kann eine beliebige Sensoranordnung zur Erfassung von wenigstens dem inspiratorischen Atemgasfluss, bevorzugt auch vom exspiratorischen Atemgasfluss, sein, wie beispielsweise ein Heißdraht-Anemometer. Bevorzugt umfasst die Flusssensoranordnung eine Druckdifferenz-Flusssensoranordnung mit einem veränderlichen Strömungswiderstand und mit beiderseits des Strömungswiderstands - bei Betrachtung längs einer Strömungsbahn des Atemgases - vorgesehenen Erfassungsorten zur Erfassung des Drucks des Atemgases. So kann mit der Flusssensoranordnung nicht nur der Atemgasfluss, sondern gleichzeitig auch der Atemgasdruck bestimmt werden. Damit die Flusssensoranordnung sowohl den Fluss und gegebenenfalls den Druck des inspiratorischen wie auch des exspiratorischen Atemgases erfassen kann, ist sie bevorzugt in dem oben genannten gemeinsam genutzten Leitungsabschnitt zwischen dem Y-Verbinder und dem Atemgaseinlass bzw. dem Atemgasauslass angeordnet.

Die Ausbildung der Beatmungsvorrichtung zur Durchführung des weiter oben beschriebenen und weitergebildeten Verfahrens zur Ermittlung der FRC ist hinsichtlich der erforderlichen Steuerungseingriffe sowie hinsichtlich der erforderlichen Datenverarbeitung durch eine entsprechende Ausbildung der Steuervorrichtung realisiert. Die Steuervorrichtung ist außerdem ausgebildet, Komponenten der Beatmungsvorrichtung derart anzusteuern, dass weiter oben definierte Verfahrensschritte an der Beatmungsvorrichtung ausgeführt werden.

Über die Möglichkeit der Veränderung des Betriebszustands von wenigstens der Druckveränderungsvorrichtung und der Steuerventilanordnung erhält die Steuervorrichtung außerdem von den genannten Sensoren Signale, aus welchen die Steuervorrichtung einen Atemgasfluss und einen Anteil des metabolisch-inerten Gases am Atemgas ermitteln kann. Dies gilt für wenigstens das inspiratorische oder das exspiratorische Atemgas, bevorzugt für das inspiratorische und das exspiratorische Atemgas.

Die Steuervorrichtung verfügt außerdem über ein Zeitsignal, sei es über eine in die Steuervorrichtungen integrierte oder eine daran angeschlossene Zeitmessvorrichtung, welche es der Steuervorrichtung ermöglicht, aus einem ermittelten Gasfluss eine während eines Zeitraums geflossene Gasmenge zu ermitteln.

Mit Verweis auf die oben zum Verfahren erläuterte sensorische Erfassung der Atemgaskomponenten weist die Gaskomponenten-Sensoranordnung bevorzugt wenigstens einen der folgenden Sensoren auf:

- einen Sauerstoffsensor zur Erfassung eines Sauerstoffgehalts im inspiratorischen und im exspiratorischen Atemgas, und
- einen Kohlendioxidsensor zur Erfassung eines Kohlendioxidgehalts im inspiratorischen und im exspiratorischen Atemgas.

Bevorzugt umfasst die Gaskomponenten-Sensoranordnung sowohl einen Sauerstoffsensor als auch einen Kohlendioxidsensor. Zur erleichterten Handhabung und zur möglichst synchronen Erfassung von Sauerstoff und Kohlendioxid im Atemgas sind der Sauerstoffsensor und der Kohlendioxidsensor vorzugsweise in einem gemeinsamen Sensorgehäuse aufgenommen. Der Kohlendioxidsensor kann ein nicht-dispersiver Infrarotsensor sein. Der Sauerstoffsensor kann ein nach dem Prinzip der Lumineszenzlöschung arbeitender Sauerstoffsensor sein.

Da die genannten Sensoren häufig nur eine physikalische Größe erfassen, welche den Partialdruck des durch den jeweiligen Sensor erfassbaren Gases repräsentieren, umfasst die Beatmungsvorrichtung bevorzugt auch ein Barometer zur Erfassung des Umgebungsluftdrucks, um aus den erfassten Größen, welche einen Partialdruck repräsentieren, einen Anteil des erfassten Gases am gesamten Atemgas zu ermitteln zu können. So ist der gemittelte tidale Anteil $^{iZp}a(n)^{tid}_{Gas}$ eines Gases am Atemgas während des n-ten Atemhubs in der i-ten Zufuhrphase:

$$^{iZp}a(n)^{tid}_{Gas} = \frac{\int_{t=_{in}t_0}^{ext_{end}} |\dot{V}| \cdot \frac{^{iZp}p^{tid}_{Gas}(t)}{^{iZp}p^{tid}_{amb}(t) + ^{iZp}p^{tid}_{awy}(t)}\, dt}{\int_{t=_{in}t_0}^{ext_{end}} |\dot{V}| \cdot dt} \qquad (Gl.\ 13)$$

mit $|\dot{V}|$ als dem Betrag des während des n-ten Atemhubs von der Flusssensoranordnung erfassten Atemgasflusses, mit $_{in}t_0$ als dem Zeitpunkt des Beginns der Inspirationsphase und mit $_{ex}t_{end}$ als dem Zeitpunkt des Endes der Exspirationsphase des n-ten Atemhubs der i-ten Zufuhrphase, und mit $^{iZp}p^{tid}_{Gas}(t)$ als dem während des n-ten Atemhubs sensorisch erfassten Partialdrucks des durch den jeweiligen Sensor erfassbaren Gases, $^{iZp}p^{tid}_{amb}(t)$ als dem während des n-ten Atemhubs erfassten Umgebungsdruck und mit $^{iZp}p^{tid}_{awy}(t)$ als dem durch vorzugsweise die Flusssensoranordnung oder/und einen gesonderten Drucksensor während des n-ten Atemhubs sensorisch erfassten Überdruck oder Unterdruck im Atemweg, insbesondere in einem proximalen Abschnitt der Beatmungsleitungsanordnung. Vorzugsweise wird der Atemwegsüber- oder -unterdruck in einem sowohl von exspiratorischem als auch von inspiratorischem Atemgas durchströmten Abschnitt der Beatmungsleitungsanordnung erfasst.

Die Steuerungsvorrichtung ist zur Ausführung der Rechenoperation der Gleichung 13 auf Grundlage der angegebenen Sensor-Erfassungswerte ausgebildet.

Im Stand der Technik ist es häufig nötig, Atemgas zur messtechnischen Erfassung für eine Bestimmung einer FRC von dem Atemgasstrom in der Beatmungsleitungsanordnung abzuzweigen und in einem gesonderten Meßzweig messtechnisch zu erfassen und zu verarbeiten. Eine derartige messtechnische Erfassung von Atemgas im sogenannten Nebenstrom erhöht das Risiko von Ungenauigkeiten in der Bestimmung der FRC. Vorrangig besteht bei der messtechnischen Erfassung von Atemgas im Nebenstrom das Problem der ausreichenden Synchronisierung der im Nebenstrom erhaltenen Messergebnisse mit dem im Hauptstrom erfolgenden Beatmungsvorgang. Das zur messtechnischen Verarbeitung in einen von einem Hauptstromabschnitt zur Patientenversorgung körperlich getrennten Nebenstromabschnitt abgezweigte Atemgas liefert zu einem Zeitpunkt Messwerte durch die sensorische Erfassung, welche von dem Zeitpunkt, zu welchem das sensorisch erfasste Atemgas der Lunge zugeführt wird oder aus dieser abströmt, versetzt sein kann. Da jedoch gemäß obiger Darstellung bevorzugt tidale Größen, also auf einen einzelnen Atemhub bezogene Größen, zur Ermittlung der funktionalen Restkapazität verwendet werden, müssen die im Nebenstromabschnitt gewonnenen Messwerte eindeutig einem Atemhub zugeordnet werden können. Da das oben beschriebene Verfahren an der vorliegend diskutierten Beatmungsvorrichtung ausgeführt werden kann, besteht dieses Synchronisierungsproblem an der Beatmungsvorrichtung nicht. Denn die Beatmungsvorrichtung ermöglicht eine sensorische Erfassung in einem Hauptstromabschnitt. Ein Hauptstromabschnitt im Sinne der vorliegenden Anmeldung ist ein Abschnitt der Beatmungsleitungsanordnung, dessen von ihm geführtes strömendes Atemgas, bevorzugt sind dies sowohl das inspiratorische wie auch das exspiratorische Atemgas, zu wenigstens 95 %-vol., vorzugsweise zu wenigstens 98 %-vol., besonders bevorzugt unter Vernachlässigung etwaiger Leckagen zu 100 %-vol. dem patientenseitigen Atemgasauslass unmittelbar zugeführt wird oder unmittelbar aus der Patientenlunge über den patientenseitigen Atemgaseinlass in die Beatmungsleitungsanordnung eingeleitet wurde. Daher ist bevorzugt vorgesehen, dass die Gaskomponenten-Sensoranordnung in einem Hauptstromabschnitt der Beatmungsleitungsanordnung zur Erfassung des Anteils des metabolisch-inerten Gases am inspiratorischen und am exspiratorischen Atemgas angeordnet ist, welcher sowohl von dem Patienten zugeführten inspiratorischen Atemgas als auch von dem vom Patienten weg strömenden exspiratorischen Atemgas durchströmt wird.

Grundsätzlich kann daran gedacht sein, die Mischvorrichtung von Hand zu betätigen, um die erste Zufuhrphase zu beenden und die zweite Zufuhrphase zu beginnen. Bevorzugt ist jedoch die Steuervorrichtung zur Ansteuerung der Mischvorrichtung ausgebildet, um durch Ansteuerung der Mischvorrichtung den Anteil an metabolisch-inertem Gas im inspiratorischen Atemgas zu verändern. Die Mischvorrichtung kann über einen Aktuator verfügen, welcher von der Steuervorrichtung ansteuerbar ist. Die Mischvorrichtung kann ein Ventil umfassen, dessen Öffnungsgrad durch die Steuervorrichtung veränderbar ist.

Die vorliegende Erfindung wird nachfolgend anhand der beiliegenden Zeichnungen näher erläutert. Es stellt dar:

Fig. 1   eine grobschematische Ansicht einer erfindungsgemäßen Beatmungsvorrichtung,

Fig. 2    eine graphische Darstellung des Verlaufs einer Tidalmengendifferenz, eines Anteils von Stickstoff im inspiratorischen Atemgas und einer Basismengendifferenz während einer zweiten Zufuhrphase, mit Darstellung des Endes einer vorhergehenden ersten Zufuhrphase und einer nachfolgenden dritten Zufuhrphase, und

Fig. 3    eine Darstellung von respiratorisch relevanten Atem- bzw. Lungenvolumina.

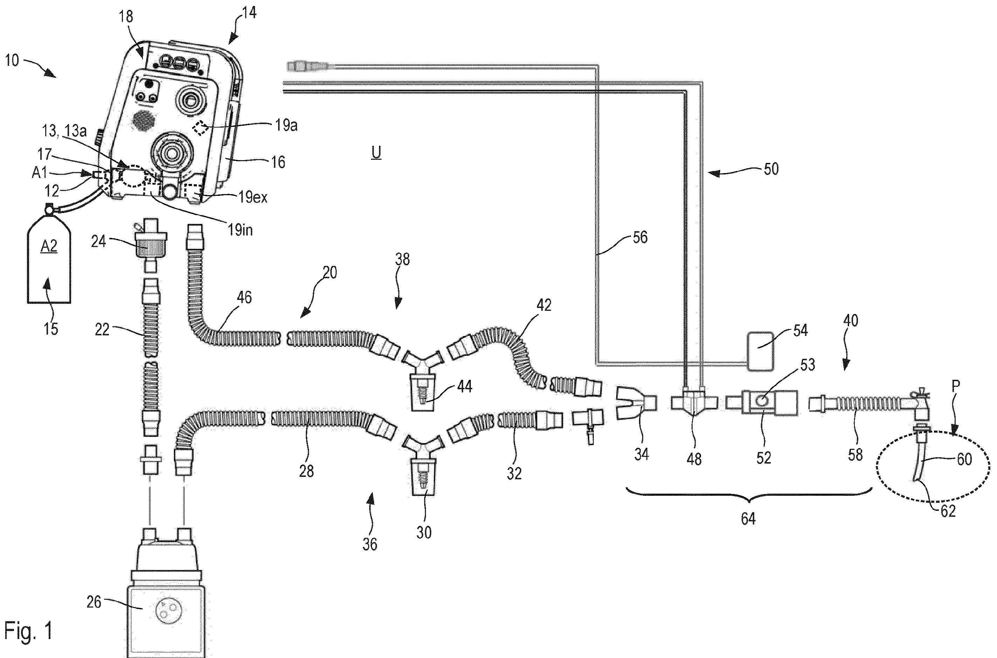

In Figur 1 ist eine erfindungsgemäße Ausführungsform einer Beatmungsvorrichtung allgemein mit 10 bezeichnet. Die Beatmungsvorrichtung 10 umfasst eine erste Atemgasquelle 12 in Gestalt eines zur Umgebung U der Beatmungsvorrichtung 10 öffnenden Ansaugstutzens. Ein leistungsveränderliches Gebläse 13, welches durch eine Steuervorrichtung 14 steuerbar ist, erlaubt Umgebungsluft als erste Atemgaskomponente A1 anzusaugen. Das Gebläse 13 und die Steuervorrichtung 14 sind im selben Gehäuse 16 aufgenommen. In diesem Gehäuse befinden sich auch an sich bekannte Ventile, wie ein Inspirationsventil 19in und ein Exspirationsventil 19ex. Die Steuervorrichtung 14 umfasst außerdem eine Zeiterfassungsvorrichtung 19a.

Außerdem ist an das Gehäuse 16 eine zweite Atemgasquelle 15 strömungsverbindend angeschlossen. Die zweite Atemgasquelle 15 kann eine unter Druck stehende Gasflasche sein, etwa mit darin bevorratetem, unter Druck stehendem reinen Sauerstoff als einer zweiten Atemgaskomponente A2.

Die an der ersten Atemgasquelle 12 angesaugte erste Atemgaskomponente A1 und die von der zweiten Atemgasquelle 15 gelieferte zweite Atemgaskomponente A2 werden zu einem Mischventil 17 geleitet, das stellungsabhängig, vorzugsweise stufenlos, aus den beiden Atemgaskomponenten ein inspiratorisches Atemgas mit einem beliebigen Mischungsverhältnis von 100 %-vol. der ersten Atemgaskomponente A1 und 0 %-vol. der zweiten Atemgaskomponente A2 bis zu 0 %-vol. der ersten Atemgaskomponente A1 und 100 %-vol. der zweiten Atemgaskomponente A2 mischt. Das Mischventil 17 und damit das Mischungsverhältnis des inspiratorischen Atemgases ist ebenfalls durch die Steuervorrichtung 14 steuerbar bzw. einstellbar.

Im dargestellten Beispiel dient $N_2$ als metabolisch-inertes Gas. Da die erste Atemgaskomponente A1 eine $N_2$-Fraktion von etwa 71 %-vol. und die zweite Atemgaskomponente eine $N_2$-Fraktion von etwa 0 %-vol. aufweist, kann das durch das Mischventil 17 gemischte inspiratorische Atemgas einen $N_2$-Anteil von zwischen 0 und 71 %-vol. aufweisen. Ein solches inspiratorisches Atemgas ist von jedem für eine künstliche Beatmung in Frage kommenden Landlebewesen dieses Planeten atembar. Bevorzugt kann die Veränderung des Mischungsverhältnisses der Atemgaskomponenten zeitlich innerhalb eines Atemhubs, besonders bevorzugt innerhalb einer Exspiration von einem ersten, früheren Mischungsverhältnis auf ein zweites, späteres Mischungsverhältnis umgestellt werden.

Die Steuervorrichtung 14 der Beatmungsvorrichtung 10 weist eine Eingabe/Ausgabeeinrichtung 18 auf, welche zahlreiche Schalter, wie Tastschalter und Drehschalter umfasst, um erforderlichenfalls Daten in die Steuervorrichtung 14 eingeben zu können. Das Gebläse 13 der ersten Atemgasquelle 12 kann in seiner Förderleistung durch die Steuervorrichtung verändert werden, um die Menge an Atemgas, das von der pro Zeiteinheit gefördert wird, zu verändern. Das Gebläse 13 ist daher im vorliegenden Ausführungsbeispiel eine Druckveränderungsvorrichtung 13a der Beatmungsvorrichtung 10.

An die vom Gebläse 13 weg und zum Patienten P hin führende Leitung ist unter Zwischenanordnung des Inspirationsventils 19in eine Beatmungsleitungsanordnung 20 angeschlossen, welche im vorliegenden Beispiel fünf flexible Schläuche umfasst. Ein erster inspiratorischer Beatmungsschlauch 22 verläuft von einem zwischen dem Inspirationsventil 19in und ihm selbst angeordneten Filter 24 zu einer optionalen Konditionierungsvorrichtung 26, wo das von der Atemgasquelle 12 gelieferte Atemgas auf einen vorgegebenen Feuchtegrad befeuchtet und gegebenenfalls mit Aerosol-Medikamenten versehen wird. Der Filter 24 filtert und reinigt die vom Gebläse 13 gelieferte Umgebungsluft.

Ein zweiter inspiratorischer Beatmungsschlauch 28 führt von der optionalen Konditionierungsvorrichtung 26 zu einer inspiratorischen Wasserfalle 30. Ein dritter inspiratorischer Beatmungsschlauch 32 führt von der Wasserfalle 30 zu einem Y-Verbinder 34, welcher die distale Inspirationsleitung 36 und die distale Exspirationsleitung 38 zu einer kombinierten proximalen inspiratorisch-exspiratorischen Beatmungsleitung 40 verbindet.

Vom Y-Verbinder 34 zurück zum Gehäuse 16 verläuft ein erster exspiratorischer Beatmungsschlauch 42 zu einer exspiratorischen Wasserfalle 44 und von dort ein zweiter exspiratorischer Beatmungsschlauch 46 zum Gehäuse 16, wo das exspiratorische Atemgas über das Exspirationsventil 19ex in die Umgebung abgelassen wird.

Auf der patientennahen kombinierten inspiratorisch-exspiratorischen Seite des Y-Verbinders 34 folgt unmittelbar auf den Y-Verbinder 34 ein Durchflusssensor 48, hier: ein Differenzdruck-Durchflusssensor 48, welcher den inspiratorischen und exspiratorischen Fluss an Atemgas zum Patienten P hin und vom Patienten P weg erfasst. Eine Leitungsanordnung 50 überträgt den beiderseits eines an sich bekannten veränderlichen Strömungshindernisses im Durchflusssensor 48 herrschenden Gasdruck an die Steuervorrichtung 14, die aus den übertragenen Gasdrücken und insbesondere aus der Differenz der Gasdrücke die Menge an pro Zeiteinheit strömendem inspiratorischem und exspiratorischem Atemgas errechnet.

In Richtung vom Y-Verbinder 34 weg folgt zum Patienten P hin auf den Durchflusssensor 48 eine Messküvette 52 sowohl zur nicht-dispersiven Infrarot-Erfassung eines vorbestimmten volumenbezogenen Gasanteils im Atemgas, hier

beispielhaft Kohlendioxid ($CO_2$), als auch zur luminiszenzbasierten Erfassung des volumenbezogenen Gasanteils von Sauerstoff ($O_2$). Dabei sind die $CO_2$-Anteile und die $O_2$-Anteile sowohl im inspiratorischen Atemgas wie auch im exspiratorischen Atemgas von Interesse, da die Änderung des $CO_2$-Anteils und des $O_2$-Anteils zwischen Inspiration und Exspiration ein Maß für die Stoffwechselfähigkeit der Patientenlunge ist. Zu erkennen ist in Figur 1 eines der seitlichen Fenster 53, durch welche Infrarotlicht in die Messküvette 52 eingestrahlt werden kann bzw. aus dieser ausstrahlen kann, je nach Orientierung eines mit der Messküvette lösbar gekoppelten kombinierten $CO_2$-$O_2$-Gassensors 54.

Der Gassensor 54 ist an die Messküvette 52 derart ankoppelbar, dass der Gassensor 54 sowohl die Messküvette 52 mit Infrarotlicht durchleuchten kann als auch eine luminophorhaltige Messfläche der Messküvette 52 zur Strahlung anregen kann.

Aus der Intensität des Infrarotlichts, genauer aus dessen spektraler Intensität kann in an sich bekannter Weise auf die Menge bzw. den Anteil eines vorbestimmten Gases in dem die Messküvette 52 durchströmenden Atemgas geschlossen werden. Das vorbestimmte Gas, hier: $CO_2$, absorbiert Infrarotlicht einer definierten Wellenlänge. Die Intensität des Infrarotlichts in dieser Wellenlänge hängt nach Durchgang im Wesentlichen von der Absorption des Infrarotlichts dieser Wellenlänge durch das vorbestimmte Gas ab. Ein Vergleich der Intensität des Infrarotlichts der definierten Wellenlänge mit einer Wellenlänge des Infrarotlichts, die zu keinem Absorptionsspektrum eines zu erwartenden Gasanteils im Atemgas gehört, liefert eine Information über den Anteil des vorbestimmten Gases am Atemgas.

Aus der vom Gassensor 54 erfassten Strahlungsantwort der luminophorhaltigen Messfläche der Messküvette 52 auf die oben beschriebene Anregung durch den Gassensor 54 hin kann unter Berücksichtigung eines Intensitätsunterschieds oder/und eines Phasenunterschieds zwischen der bevorzugt modulierten Anregungsstrahlung und angeregter Strahlung der volumenbezogene $O_2$-Anteil am Atemgas ermittelt werden. O2 wirkt als Quenchersubstanz für das Luminophor der Messfläche und beeinflusst die Antwortstrahlung hinsichtlich Intensität oder/und Phasenverschiebung maßgeblich.

Der Gassensor 54 ist daher über eine Datenleitung 56 mit der Steuervorrichtung 14 der Beatmungsvorrichtung 10 verbunden und überträgt über die Datenleitung 56 die beschriebenen Intensitätsinformationen an die Steuervorrichtung 14.

Auf die Messküvette 52 folgt in Richtung zum Patienten P hin ein weiteres Schlauchstück 58, an welchen ein Endotrachealtubus 60 als Beatmungsschnittstelle zum Patienten P angeordnet ist. Eine proximale Öffnung 62 des Endotrachealtubus 60 ist sowohl Atemgas-Auslassöffnung, durch welche inspiratorisches Atemgas durch den Endotrachealtubus 60 in den Patienten P eingeleitet wird, als auch Atemgas-Einlassöffnung, durch welche exspiratorisches Atemgas aus dem Patienten zurück in den Endotrachealtubus 60 geleitet wird.

Die gesamte Beatmungsleitungsanordnung ist eine Hauptstromleitung, ohne Abzweigung einer Nebenstromleitung davon. Der proximale einstrangige Abschnitt des Y-Verbinders 34, die Durchflusssensor 48, die Messküvette 52 und das Schlauchstück 58 bilden einen außerhalb des Körpers des Patienten P gelegenen Hauptstromabschnitt 64, welcher sowohl von inspiratorischem als auch von exspiratorischem Atemgas durchströmt wird.

Die Steuervorrichtung 14 ist dazu ausgebildet, das Gebläse 13 und die Mischvorrichtung 17 gemäß dem eingangs beschriebenen Verfahren anzusteuern, um aus den Erfassungswerten, die mit dem Gassensor 54 und mit dem Durchflusssensor 48 erfasst werden, eine funktionalen Restkapazität FRC der Lunge des Patienten P zu ermitteln.

Hierzu wird dem Patienten P in einer ersten Zufuhrphase 70 zunächst ein erstes inspiratorisches Atemgas zugeführt, welches gebildet ist aus einer Mischung der beiden Atemgaskomponenten A1 und A2, sodass das inspiratorische Atemgas einen höheren Sauerstoffanteil aufweist als die erste Atemgaskomponente A1, also Umgebungsluft, für sich alleine genommen.

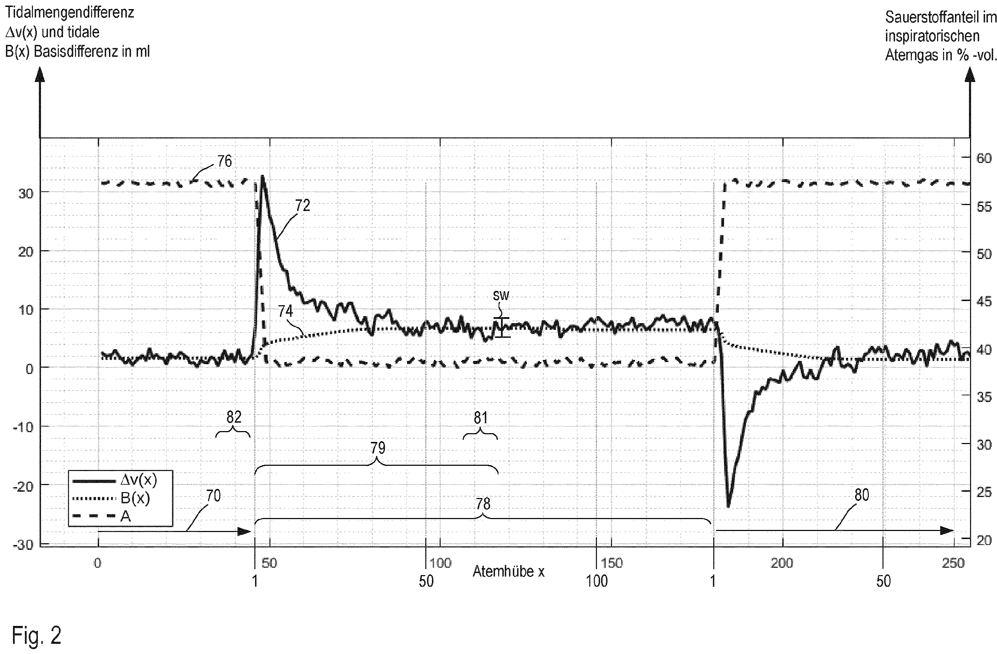

Das Ende dieser ersten Zufuhrphase 70 ist in dem Diagramm von Figur 2 mit Pfeil gekennzeichnet.

Figur 2 zeigt drei Diagramme und weist dafür zwei Skalen auf. Die linke Ordinaten-Skala in Figur 2 bezieht sich auf Tidalmengendifferenzen in Millilitern und gilt für die in durchgezogener Linie dargestellte und mit Bezugszeichen 72 bezeichnete Tidalmengendifferenz ${}^{iZp}\Delta v(x)^{tid}_{N_2}$, oder in Fig. 2 kurz $\Delta v(x)$, wie sie aus den vom Gassensor 54 erfassten Werten mittels Gleichung 1 für jeden Atemhub bestimmt wird. Sie gilt außerdem für die mit gepunkteter Linie dargestellte und mit Bezugszeichen 74 bezeichnete tidale Basisdifferenz ${}^{iZp}B^{tid}(x)$, oder in Fig. 2 kurz $B(x)$, wie sie gemäß Gleichung 6 ermittelt wird.

Die rechte Ordinaten-Skala in Figur 2 bezieht sich auf den Anteil ${}^{iZp}_{in}A_{O_2}$, oder in Fig. 2 kurz A, an Sauerstoff im inspiratorischen Atemgas in Volumenprozent. Der Graph des Sauerstoffanteils im inspiratorischen Atemgas ist in Figur zwei strichliniert dargestellt und mit Bezugszeichen 76 bezeichnet.

Die Abszisse der Darstellung von Figur 2 gibt die Atemhübe x an. Die Abszisse hat dabei zwei Skalen, von welchen eine bei null beginnt und für jeden Atemhub im betrachteten Zeitraum um den Wert 1 inkrementiert. Die andere Skala inkrementiert ebenfalls um den Wert 1, beginnt jedoch bei jeder Zufuhrphase erneut mit dem Wert 1 zu zählen.

Da dieses erste inspiratorische Atemgas in der ersten Zufuhrphase 70 wegen der stärkeren Zumischung der zweiten Atemgaskomponente A2 eine geringere Menge bzw. einen geringeren Mengenanteil an Stickstoff als dem metabolisch-inerten Gas als das zweite inspiratorische Atemgas enthält, entspricht die erste Zufuhrphase 70 einer in der Beschreibungseinleitung beschriebenen Auswaschphase bzw. "Wash-Out"-Phase.

Dabei wird tidal, also für jeden Atemhub, die Zusammensetzung des ersten inspiratorischen Atemgases sowie des aus diesem gebildeten ersten exspiratorischen Atemgases erfasst. Aus der vom Durchflusssensor 48 erhaltenen Flussinformation als pro Zeiteinheit inspiratorisch und exspiratorisch geströmtem Atemgasvolumen, und aus den vom Gassensor 54 erhaltenen Volumenanteilen an Sauerstoff und Kohlendioxid sowohl am inspiratorischen Atemgas als auch am exspiratorischen Atemgas können für jeden Atemhub sowohl die inspiratorisch verabreichten Mengen wie auch die exspiratorisch ausgeschiedenen Mengen an Sauerstoff, Kohlendioxid und Stickstoff unter der vereinfachenden aber ausreichend genauen Annahme erhalten werden, dass das inspiratorische und das exspiratorischen Atemgas über Sauerstoff, Kohlendioxid und Stickstoff hinaus keine weiteren Bestandteile in signifikantem Umfang enthalten.

Somit können die Tidalmengendifferenzen gemäß Gleichung 1 unmittelbar aus den für die Steuervorrichtung 14 verfügbaren Erfassungsergebnissen ermittelt werden. Mit den Tidalmengendifferenzen lässt sich auch deren gleitender arithmetischer Mittelwert gemäß Gleichung 2 durch die Steuervorrichtung 14 ermitteln. Ebenso wird der gemittelte Anteil an Stickstoff im ersten inspiratorischen Atemgas gemäß Gleichung 8 oder 8a ermittelt. Die ermittelten Werte werden in einem Datenspeicher der Steuervorrichtung 14 abgespeichert.

Liegt der gleitend gemittelte Differenzenwert gemäß Gleichung 2 für die erste Zufuhrphase 70 betragsmäßig unter einem vorbestimmten Schwellenwert oder ist diesem gleich, so beendet die Steuervorrichtung 14 die erste Zufuhrphase durch Verstellen des Mischventils als der Mischvorrichtung 17 und führt dem Patienten P ein zweites inspiratorisches Atemgas zu, dessen Stickstoffanteil gegenüber dem ersten inspiratorischen Atemgas verändert, im vorliegenden Beispiel erhöht ist. Es beginnt also die zweite Zufuhrphase 78, zu erkennen in Figur 2 an dem linken Wert 1 in der unteren Abszissen-Skala. Die zweite Zufuhrphase 78 dauert für etwa 130 Atemhübe an.

Die Menge an der Atemgaskomponente A2, welche der Atemgaskomponente A1 zugemischt wird, ist in der zweiten Zufuhrphase 78 geringer als in der ersten Zufuhrphase 70. Wegen der Verstellung des Mischventils 17 fällt der Sauerstoffanteil im Atemgas schlagartig von etwa 57 %-vol. auf etwa 38 %-vol. ab. Der Sauerstoffanteil liegt jedoch immer noch höher als bei reiner Umgebungsluft.

Mit der zweiten Zufuhrphase 78 beginnt der Ermittlungszeitraum über welchen hinweg die FRC ermittelt wird. Dabei muss die FRC nicht in Echtzeit während der zweiten Zufuhrphase ermittelt werden, sondern es müssen lediglich die zur Ermittlung der FRC verwendeten Tidalmengendifferenzen dem Ermittlungszeitraum entstammen.

Ab Beginn der zweiten Zufuhrphase wird daher für jeden Atemhub eine Tidalmengendifferenz gemäß Gleichung 1 berechnet. Sofern bereits ausreichend viele Atemhübe zur Mittelwertbildung erfolgt sind, wird auch der gleitende Mittelwert der Tidalmengendifferenzen gemäß Gleichung 2 gebildet. Wiederum gilt: sinkt der gleitende Differenzenwert gemäß Gleichung 2 auf oder unter einen hierfür vorbestimmten Schwellenwert, endet der Ermittlungszeitraum.

Der Verlauf der Tidalmengendifferenz über den beobachteten Zeitraum ist durch den Graphen 72 angegeben. Da sich aufgrund von Totvolumina in der Patientenlunge in der Patientenlunge noch Atemgas der ersten Zufuhrphase mit geringerem Stickstoffanteil befindet, wird vom Patienten P zunächst ab Beginn der zweiten Zufuhrphase 78 zweites exspiratorisches Atemgas ausgeatmet, welches einen höheren Volumenanteil an Stickstoff aufweist als das zweite inspiratorische Atemgas. Die Tidalmengendifferenz für die Atemhübe zu Beginn der zweiten Zufuhrphase 78 ist daher positiv und weicht signifikant sowohl von dem Wert 0 ab, welcher dann erreicht wird, wenn das exspiratorische und das inspiratorische Atemgas einen gleich großen Stickstoffanteil aufweisen. Die Tidalmengendifferenz 72 weicht jedoch auch signifikant von der Basisdifferenz 74 gegen Ende der ersten Zufuhrphase 70 ab. Damit der zweiten Zufuhrphase 78 das Auswaschen von Stickstoff aus der Patientenlunge erfolgt nimmt die Tidalmengendifferenz 72 mit zunehmenden Abstand vom Beginn der zweiten Zufuhrphase 78 betragsmäßig ab, bis sie sich um einen konstanten Offset-Wert ab etwa dem 50. Atemhub der zweiten Zufuhrphase 78 einpendelt. Ab etwa diesem 50. Atemhub der zweiten Zufuhrphase 78 ändert sich die Tidalmengendifferenz 72 nicht mehr substantiell, sondern wird im Wesentlichen nur noch von Störeinflüssen der Beatmungsvorrichtung 10, wie beispielsweise Leckagen und dergleichen, beeinflusst.

Die tidale Basisdifferenz 74 wird, wie oben bereits erwähnt, gemäß obiger Gleichung 6 ermittelt. Sie steigt ausgehend von dem Wert der tidalen Basisdifferenz 74 gegen Ende der ersten Zufuhrphase 70 zunächst stark, dann immer schwächer an, bis sie im Wesentlichen zu dem Offset-Wert der Tidalmengendifferenz 72 konvergiert.

Nach Ende des Ermittlungszeitraums wird der Anteil an Stickstoff im zweiten inspiratorischen Atemgas gemäß Gleichung 8 oder 8a ermittelt. Bei Verwendung der Gleichung 8a sollte die Konstante p größer als 100 gewählt sein und sollte die Konstante q nicht größer als 50 gewählt sein, sodass die zur Anwendung der Gleichung 8a verwendeten Erfassungswerte jenem Bereich, etwa dem End-Erfassungsbereich 81 in der zweiten Zufuhrphase 78 entstammen, in welchem die Tidalmengendifferenz 72 einen im Wesentlichen konstanten bzw. um einen konstanten Wert pendelnden Wert aufweist.

Die FRC wird dann bevorzugt aus Gleichung 11b berechnet, um die FRC mit hoher Genauigkeit zu erhalten. Alternativ könnte jedoch auch die Gleichung 11a herangezogen werden.

Da die Basisdifferenz am Ende der ersten Zufuhrphase 70 ermittelbar ist, bevor die zweite Zufuhrphase 78 beginnt, und da der Stickstoffanteil im inspiratorischen Atemgas der zweiten Zufuhrphase 78 zumindest als Sollwert bekannt ist, kann unter Verwendung des Sollwerts für den Stickstoffanteil der zweiten Zuführphase 78 die FRC während einer künstlichen Beatmung des Patienten P sogar in Echtzeit ermittelt werden. Denn dann sind zum Zeitpunkt eines jeden Atemhubs der zweiten Zuführphase alle zur Berechnung von Gleichung 11b benötigten Daten bekannt.

Wenngleich die gesamte zweite Zuführphase 78 zur Ermittlung der FRC herangezogen werden kann, reicht ein kürzerer Ermittlungszeitraum 79 aus. Bevorzugt reicht es aus, wenn der Ermittlungszeitraum 79 gemeinsam mit der zweiten Zuführphase 78 beginnt, und wenn er in dem Bereich endet, in welchem sich die tidale Mengendifferenz 72 und die tidale Basisdifferenz um nicht mehr als einen vorbestimmten kleinen Schwellenwert sw betragsmäßig unterscheiden.

In Figur 2 ist der Beginn einer dritten Zuführphase 80 dargestellt, mit welcher wieder erstes Atemgas mit höherem Sauerstoffanteil dem Patienten P zugeführt wird. Folglich wird aus dessen Lunge aufgrund von den Todräumen der Lunge noch vorhandener Stickstoff ausgewaschen, sodass das exspiratorische Atemgas einen höheren Stickstoffanteil aufweist als das inspiratorische Atemgas desselben Atemhubs. Somit ist die Tidalmengendifferenz negativ und nimmt betragsmäßig mit zunehmenden Abstand vom Beginn der dritten Zuführphase 80 ab. Da in der ersten und der dritten Zuführphase 70 bzw. 80 jeweils das gleiche erste inspiratorische Atemgas verwendet wird, stellen sich mit Fortdauer der dritten Zuführphase 80 die gleichen Verhältnisse ein wie gegen Ende der ersten Zuführphase 70.

Alternativ kann die FRC anstelle von Gleichung 11b auch gemäß Gleichung 11a berechnet werden, wobei hierzu die Basisdifferenz B als Mittelwert der Tidalmengendifferenz $\Delta v(x)$ über einen in der zweiten Zuführphase 78 gelegenen End-Erfassungsabschnitt 81 oder, wegen der niedrigeren erzielbaren Genauigkeit der FRC-Ermittlung jedoch weniger bevorzugt, über einen der ersten Zuführphase 70 gelegenen Anfangs-Erfassungsabschnitt 82 berechnet wird. Der End-Erfassungsabschnitt 81 liegt dem Ende des Erfassungszeitraums 79 näher als dessen Beginn bzw. als dem gleichzeitigen Beginn der zweiten Zuführphase 78. Der Anfangs-Erfassungsabschnitt 82 liegt dem Anfang der zweiten Zuführphase 78 näher als dem Anfang der ersten Zuführphase 70.

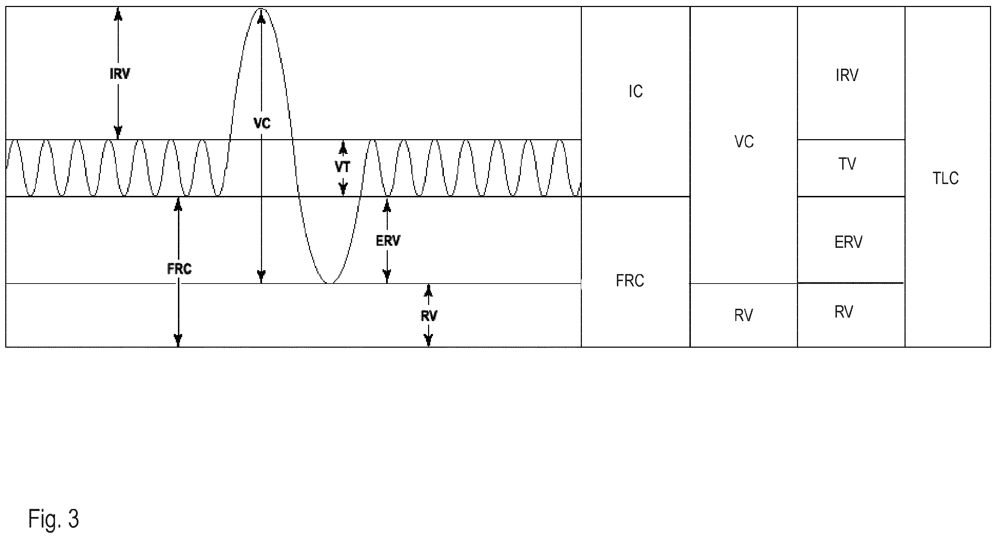

In Figur 3, welche zurückgeht auf die Quelle "Vihsadas" in en.wikipedia, sind die unterschiedlichen Teilvolumina einer Patientenlunge erläutert, um deutlich zu machen, was genau mit funktionaler Restkapazität gemäß der vorliegenden Anmeldung gemeint ist.

In Figur 3 links ist ein spirometrischer Verlauf eines Atemgasvolumens in einer Patientenlunge über mehrere Atemhübe hinweg aufgezeichnet.

Eine totale Lungenkapazität TLC ist jenes Volumen, welches theoretisch ausgehend von einer vollständig kollabierten Patientenlunge bis zum maximal möglichen Einatmen in eine Lunge einleitbar ist. Dieser Wert ist rein theoretisch, da eine vollständig kollabierte Patientenlunge für den Patienten P letal wäre.

Es verbleibt daher in einer funktionierenden Patientenlunge stets ein Restvolumen RV, das der Patient P selbst bei größter Anstrengung nicht aus seiner Lunge austreiben kann. Die vitale Kapazität VC der Patientenlunge ist jenes Volumen, das der Patient P zwischen einem mit maximaler Anstrengung maximal ausgeatmeten Zustand und einem mit maximaler Anstrengung maximal eingeatmeten Zustand seiner Lunge zuführen bzw. aus seiner Lunge abführen kann.

Bei herkömmlichem, im Wesentlichen anstrengungslosem Atmen wird der Patientenlunge P das Tidalvolumen TV zugeführt und wieder aus dieser abgeführt. Atmet der Patient P von einem anstrengungslos ausgeatmeten Zustand ausgehend maximal ein, führt er seiner Lunge die sogenannte Inspirationskapazität IC an Atemgas zu. Atmet er ausgehend von einem anstrengungslos eingeatmeten Zustand unter Aufbietung seiner gesamten inspiratorischen Kraft maximal ein, so füllt er seine Lunge zusätzlich mit dem inspiratorischen Reservevolumen die IRV. Atmet er ausgehend von einem anstrengungslos ausgeatmeten Zustand unter Aufbietung seiner gesamten exspiratorischen Kraft maximal aus, atmet der Patient P dabei das exspiratorische Reservevolumen ERV seiner Lunge aus.

Die Summe aus Restvolumen RV und vitaler Kapazität VC entspricht ebenso der totalen Lungenkapazität TLC wie die Summe aus Restvolumen RV, exspiratorischem Reservevolumen ERV und inspiratorischer Kapazität IC.

Die Differenz totale Lungenkapazität TLC und der inspiratorischen Kapazität IC ist die funktionale Restkapazität FRC der Patientenlunge. Diese ergibt sich ebenso aus der Summe aus Restvolumen und exspiratorischem Reservevolumen ERV. Ebenso ist die funktionale Restkapazität FRC die totale Lungenkapazität TLC abzüglich des Tidalvolumens TV und weiter abzüglich des inspiratorischen Reservevolumens IRV.

**Patentansprüche**

1. Beatmungsvorrichtung (10), welche sowohl zur wenigstens teilweisen künstlichen Beatmung von lebenden Patienten (P) als auch zur Durchführung eines Verfahrens zur Ermittlung einer funktionalen Restkapazität (FRC) einer Patientenlunge ausgebildet ist, wobei das Verfahren zur Ermittlung einer funktionalen Restkapazität (FRC) einer Patientenlunge die folgenden Merkmale umfasst:

    - Zuführen eines ersten inspiratorischen Atemgases mit einem ersten Anteil an einem metabolisch-inerten Gas während einer ersten zeitlichen Zuführphase (70),
    - nach der ersten Zuführphase (70): Zuführen eines vom ersten verschiedenen zweiten inspiratorischen Atemgases mit einem vom ersten verschiedenen zweiten Anteil an dem metabolisch-inerten Gas während einer zweiten zeitlichen Zufuhrphase (78),

- Ermitteln einer während der zweiten Zufuhrphase (78) auftretenden Mengendifferenz, welche für einen Ermittlungszeitraum eine Unterschiedsmenge repräsentiert zwischen einer Menge an inspiratorischem und einer Menge an exspiratorischem metabolisch-inertem Gas, wobei der Ermittlungszeitraum nicht nach der zweiten Zufuhrphase endet,

- Ermitteln der funktionalen Restkapazität (FRC) unter Berücksichtigung der Mengendifferenz und einer Anteilsdifferenz zwischen einer ersten Anteilsgröße, welche den ersten Anteil an dem metabolisch-inerten Gas im ersten inspiratorischen Arbeitsgas repräsentiert und einer zweiten Anteilsgröße, welche den zweiten Anteil an dem metabolisch-inerten Gas im zweiten inspiratorischen Arbeitsgas repräsentiert,

- Ermitteln einer Basisdifferenz (74), welche eine Differenz zwischen einer tidalen Menge an inspiratorischem metabolisch-inerten Gas und einer tidalen Menge an exspiratorischem metabolisch-inerten Gas in der ersten oder/und der zweiten Zufuhrphase (78) repräsentiert,

wobei das Ermitteln der funktionalen Restkapazität (FRC) auf Grundlage einer korrigierten Mengendifferenz und der Anteilsdifferenz erfolgt, wobei die korrigierte Mengendifferenz durch Berücksichtigung der Basisdifferenz bei der Ermittlung der Mengendifferenz gebildet wird,

wobei die Beatmungsvorrichtung (10) umfasst:

- eine erste Atemgasquelle (12), welche eine erste inspiratorische Atemgaskomponente (A1) mit einer ersten Fraktion des metabolisch-inerten Gases bereitstellt,
- eine zweite Atemgasquelle (15), welche eine zweite inspiratorische Atemgaskomponente (A2) mit einer von der ersten verschiedenen zweiten Fraktion des metabolisch-inerten Gases bereitstellt,
- eine veränderlich einstellbare Mischvorrichtung (17) zur Bildung eines inspiratorischen Atemgases mit veränderlichem Anteil an metabolisch-inertem Gas aus der ersten oder/und der zweiten inspiratorischen Atemgaskomponente (A1, A2),
- eine Beatmungsleitungsanordnung (20) zur Förderung des inspiratorischen Atemgases zu einem patientenseitigen Atemgasauslass (62) hin und zur Förderung von exspiratorischem Atemgas von einem patientenseitigen Atemgaseinlass (62) weg,
- eine Steuerventilanordnung, umfassend ein Inspirationsventil (19in) und ein Exspirationsventil (19ex),
- eine Druckveränderungsvorrichtung (13) zur Veränderung wenigstens des inspiratorischen Atemgases in der Beatmungsleitungsanordnung (20),
- eine Flusssensoranordnung (48) zur Erfassung wenigstens des inspiratorischen Atemgasflusses,
- eine Gaskomponenten-Sensoranordnung (52, 54) zur mittelbaren oder unmittelbaren Erfassung des Anteils des metabolisch-inerten Gases am inspiratorischen und am exspiratorischen Atemgas,
- eine Steuervorrichtung (14), welche zur Steuerung der Steuerventilanordnung und der Druckveränderungsvorrichtung (13) ausgebildet ist und welche signalübertragungsmäßig mit der Flusssensoranordnung (48) und der Gaskomponenten-Sensoranordnung (52, 54) zur Übertragung von jeweiligen Erfassungssignalen an die Steuervorrichtung (14) verbunden ist.

2. Beatmungsvorrichtung (10) nach Anspruch 1,
**dadurch gekennzeichnet, dass** die Gaskomponenten-Sensoranordnung (52, 54) wenigstens einen der folgenden Sensoren aufweist:

- einen Sauerstoffsensor zur Erfassung eines Sauerstoffgehalts im inspiratorischen und im exspiratorischen Atemgas, und
- einen Kohlendioxidsensor zur Erfassung eines Kohlendioxidgehalts im inspiratorischen und im exspiratorischen Atemgas.

3. Beatmungsvorrichtung nach Anspruch 1 oder 2,
**dadurch gekennzeichnet, dass** die Gaskomponenten-Sensoranordnung (52, 54) in einem Hauptstromabschnitt (64) der Beatmungsleitungsanordnung (20) zur Erfassung des Anteils des metabolisch-inerten Gases am inspiratorischen und am exspiratorischen Atemgas angeordnet ist, welcher sowohl von dem Patienten (P) zugeführten inspiratorischen Atemgas als auch von dem vom Patienten (P) weg strömenden exspiratorischen Atemgas durchströmt wird.

4. Beatmungsvorrichtung (10) nach einem der Ansprüche 1 bis 3,
**dadurch gekennzeichnet, dass** die Steuervorrichtung (14) zur Ansteuerung der Mischvorrichtung (17) ausgebildet ist, um durch Ansteuerung der Mischvorrichtung (17) den Anteil an metabolisch-inertem Gas im inspiratorischen Atemgas zu verändern.

5. Beatmungsvorrichtung (10) nach einem der vorhergehenden Ansprüche,
**dadurch gekennzeichnet, dass** die Basisdifferenz (74) wenigstens einen Mittelwert aus einer Mehrzahl von Tidalmengendifferenzen zwischen jeweils einer tidalen Menge an inspiratorischem metabolisch-inerten Gas und einer tidalen Menge an exspiratorischem metabolisch-inerten Gas für eine Mehrzahl von Atemhüben in der ersten oder/und der zweiten Zufuhrphase (78) umfasst.

6. Beatmungsvorrichtung (10) nach einem der vorhergehenden Ansprüche,

**dadurch gekennzeichnet, dass** die Basisdifferenz (74) einen Mittelwert aus einer Mehrzahl von Tidalmengendifferenzen zwischen jeweils einer tidalen Menge an inspiratorischem metabolisch-inerten Gas und einer tidalen Menge an exspiratorischem metabolisch-inerten Gas für eine Mehrzahl von Atemhüben in einem in der ersten Zufuhrphase (70) gelegenen zeitlichen Anfangs-Erfassungsabschnitt (82) umfasst, wobei der Anfangs-Erfassungsabschnitt (82) dem Anfang der zweiten Zufuhrphase (78) näher gelegen ist als dem Anfang der ersten Zufuhrphase (70),
oder/und
dass die Basisdifferenz (74) einen Mittelwert aus einer Mehrzahl von Tidalmengendifferenzen zwischen jeweils einer tidalen Menge an inspiratorischem metabolisch-inerten Gas und einer tidalen Menge an exspiratorischem metabolisch-inerten Gas für eine Mehrzahl von Atemhüben in einem in der zweiten Zufuhrphase (78) gelegenen zeitlichen End-Erfassungsabschnitt (81) umfasst, wobei der End-Erfassungsabschnitt (81) dem Ende der zweiten Zufuhrphase (78) näher gelegen ist als deren Anfang.

7. Beatmungsvorrichtung (10) nach einem der vorhergehenden Ansprüche,
**dadurch gekennzeichnet, dass** die Basisdifferenz (74) zumindest während eines Abschnitts in der zweiten Zufuhrphase (78) oder/und im Erfassungszeitraum (79) eine tidale Basisdifferenz (74) umfasst, welche für einen Atemhub in Abhängigkeit von einem Anteil des metabolisch-inerten Gases am Atemgas des jeweiligen Atemhubs bestimmt wird.

8. Beatmungsvorrichtung (10) nach einem der vorhergehenden Ansprüche,
**dadurch gekennzeichnet, dass** die korrigierte Mengendifferenz einer Summe von korrigierten Tidalmengendifferenzen über eine Anzahl an Atemhüben im Ermittlungszeitraum entspricht, wobei die Beatmungsvorrichtung (10) dazu ausgebildet ist, für jeden Atemhub eine korrigierte Tidalmengendifferenz zu bilden aus einer Differenz einer Tidalmengendifferenz dieses Atemhubs und einer dem Atemhub zugeordneten Basisdifferenz, wobei die Beatmungsvorrichtung (10) dazu ausgebildet ist, für jeden Atemhub die Tidalmengendifferenz zu bilden durch die Differenz zwischen einer tidalen Menge an inspiratorischem metabolisch-inerten Gas und einer tidalen Menge an inspiratorischem metabolisch-inerten Gas dieses Atemhubs.

9. Beatmungsvorrichtung (10) nach einem der vorhergehenden Ansprüche,

**dadurch gekennzeichnet, dass** die erste Anteilsgröße einen über eine Mehrzahl von Atemhüben in der ersten Zufuhrphase hinweg gebildeten Mittelwert des ersten Anteils an dem metabolisch-inerten Gas im ersten inspiratorischen oder exspiratorischen Arbeitsgas umfasst oder ist,
oder/und
dass die zweite Anteilsgröße einen über eine Mehrzahl von Atemhüben in der zweiten Zufuhrphase hinweg gebildeten Mittelwert des zweiten Anteils an dem metabolisch-inerten Gas im zweiten inspiratorischen oder exspiratorischen Arbeitsgas umfasst oder ist.

10. Beatmungsvorrichtung (10) nach Anspruch 9,

**dadurch gekennzeichnet, dass** die Mehrzahl von Atemhüben, über welche hinweg die erste Anteilsgröße als Mittelwert ermittelt wird, näher am Anfang des Ermittlungszeitraums oder/und der zweiten Zufuhrphase gelegen ist als am Anfang der ersten Zufuhrphase,
oder/und
die Mehrzahl von Atemhüben, über welche hinweg die zweite Anteilsgröße als Mittelwert ermittelt wird, näher am Ende des Ermittlungszeitraums oder/und der zweiten Zufuhrphase gelegen ist als am Anfang des Ermittlungszeitraums bzw. der zweiten Zufuhrphase.

**11.** Beatmungsvorrichtung (10) nach einem der vorhergehenden Ansprüche,
**dadurch gekennzeichnet, dass** das Ermitteln der funktionalen Restkapazität auf Grundlage eines Quotienten aus der korrigierten Mengendifferenz und der Anteilsdifferenz erfolgt.

**12.** Beatmungsvorrichtung (10) nach einem der vorhergehenden Ansprüche,
**dadurch gekennzeichnet, dass** am Ende einer Mehrzahl von Atemhüben während der zweiten Zufuhrphase am Ende einer Exspirationsphase ein Atemwegsdruck im Atemweg des Patienten oder/und in einem proximalen Bereich einer Beatmungsleitung der PEEP ist.

**13.** Beatmungsvorrichtung (10) nach einem der vorhergehenden Ansprüche,
**dadurch gekennzeichnet, dass** die Beatmungsvorrichtung (10) sowohl einen inspiratorischen Atemgasstrom als auch einen exspiratorischen Atemgasstrom sensorisch erfasst.

**14.** Beatmungsvorrichtung (10) nach einem der vorhergehenden Ansprüche,
**dadurch gekennzeichnet, dass** die Beatmungsvorrichtung (10) dazu ausgebildet ist, das Verfahren zur Ermittlung einer funktionalen Restkapazität (FRC) einer Patientenlunge während einer künstlichen Beatmung eines Patienten durchzuführen.

**Claims**

**1.** A ventilator (10), which is designed both for the at least partial artificial respiration of living patients (P) as well as for carrying out a method for ascertaining a functional residual capacity (FRC) of a lung of a patient, wherein the method for ascertaining a functional residual capacity (FRC) of a lung of a patient comprises the following steps:,

- supplying a first inspiratory respiratory gas having a first proportion of a metabolically inert gas during a first temporal supply phase (70),
- following the first supply phase (70): supplying a second inspiratory respiratory gas, differing from the first and having a second proportion of the metabolically inert gas differing from the first, during a second temporal supply phase (78),
- ascertaining a difference in amount occurring during the second supply phase (78), which represents a difference amount for an ascertainment period between an amount of inspiratory metabolically inert gas and an amount of expiratory metabolically inert gas, the ascertainment period not ending after the second supply phase,
- ascertaining the functional residual capacity (FRC) by taking into account the difference in amount and a difference in proportion between a first proportion quantity, which represents the first proportion of the metabolically inert gas in the first inspiratory working gas, and a second proportion quantity, which represents the second proportion of the metabolically inert gas in the second inspiratory working gas.

**characterized in that** the method comprises the following further steps:

- ascertaining a base difference (74), which represents a difference between a tidal amount of inspiratory metabolically inert gas and a tidal amount of expiratory metabolically inert gas in the first and/or the second supply phase (78),
Wherein the ascertainment of the functional residual capacity (FRC) occurs on the basis of a corrected difference in amount and the difference in proportion, the corrected difference in amount being formed by taking into account the base difference when ascertaining the difference in amount,
wherein the ventilator (10) comprises:

- a first respiratory gas source (12), which provides a first inspiratory respiratory gas component (A1) having a first fraction of the metabolically inert gas,
- a second respiratory gas source (15), which provides a second inspiratory respiratory gas component (A2) having a second fraction of the metabolically inert gas differing from the first fraction,
- a variably settable mixing device (17) for forming an inspiratory respiratory gas having a variable proportion of metabolically inert gas from the first and/or the second inspiratory respiratory gas component (A1, A2),
- a ventilation line system (20) for conveying the inspiratory respiratory gas to a patient-side respiratory gas outlet (62) and for conveying expiratory respiratory gas away from a patient-side respiratory gas inlet (62),
- a control valve system, comprising an inspiration valve (19in) and an expiration valve (19ex),

- a pressure changing device (13) for changing at least the inspiratory respiratory gas in the ventilation line system (20),
- a flow sensor system (48) for detecting at least the inspiratory respiratory gas flow,
- a gas component sensor system (52, 54) for the indirect or direct detection of the proportion of the metabolically inert gas in the inspiratory and in the expiratory respiratory gas,
- a control device (14), which is designed to control the control valve system and the pressure changing device (13) and which is connected in signal-transmitting fashion to the flow sensor system (48) and to the gas component sensor system (52, 54) for transmitting respective detection signals to the control device (14).

2. The ventilator (10) as recited in Claim 1,
**characterized in that** the gas component sensor system (52, 54) comprises at least one of the following sensors:

- an oxygen sensor for detecting an oxygen content in the inspiratory and in the expiratory respiratory gas, and
- a carbon dioxide sensor for detecting a carbon dioxide content in the inspiratory and in the expiratory respiratory gas.

3. The ventilator as recited in Claim 1 or 2,
**characterized in that** the gas component sensor system (52, 54) is situated in a main flow section (64) of the ventilation line system (20) for detecting the proportion of the metabolically inert gas in the inspiratory and in the expiratory respiratory gas, through which both the inspiratory respiratory gas fed to the patient (P) as well as the expiratory respiratory gas flowing away from the patient (P) flow.

4. The ventilator (10) as recited in one of Claims 1 through 3,
**characterized in that** the control device (14) is designed for controlling the mixing device (17) so as to change the proportion of metabolically inert gas in the inspiratory respiratory gas by controlling the mixing device (17).

5. The ventilator (10) as recited in one of the preceding claims,
**characterized in that** the base difference (74) comprises at least one average value from a plurality of differences in tidal amounts between respectively a tidal amount of inspiratory metabolically inert gas and a tidal amount of expiratory metabolically inert gas for a plurality of breaths in the first and/or the second supply phase (78).

6. The ventilator (10) as recited in one of the preceding claims,

**characterized in that** the base difference (74) comprises an average value from a plurality of differences in tidal amounts between respectively a tidal amount of inspiratory metabolically inert gas and a tidal amount of expiratory metabolically inert gas for a plurality of breaths in a temporal start detection segment (82) in the first supply phase (70), the start detection segment (82) being closer to the start of the second supply phase (78) than to the start of the first supply phase (70),
and/or
**in that** the base difference (74) comprises an average value from a plurality of differences in tidal amounts between respectively a tidal amount of inspiratory metabolically inert gas and a tidal amount of expiratory metabolically inert gas for a plurality of breaths in a temporal end detection segment (81) in the second supply phase (78), the end detection segment (81) being closer to the end of the second supply phase (78) than to its start,

7. The ventilator (10) as recited in one of the preceding claims,
**characterized in that** the base difference (74) comprises at least during a segment in the second supply phase (78) and/or in the detection period (79) a tidal base difference (74), which is determined for a breath depending on a proportion of the metabolically inert gas in the respiratory gas of the respective breath.

8. The ventilator (10) as recited in one of the preceding claims,
**characterized in that** the corrected difference in amount corresponds to a sum of corrected differences in tidal amounts over a number of breaths in the ascertainment period, wherein the ventilator (10) is configured to form a corrected difference in the tidal amounts for every breath from a difference of a difference in the tidal amounts of this breath and a base difference associated with the breath, wherein the ventilator (10) is configured to form the difference in the tidal amounts for every breath by the difference between a tidal amount of inspiratory metabolically inert gas and a tidal amount of inspiratory metabolically inert gas of this breath.

**9.** The ventilator (10) as recited in one of the preceding claims,

**characterized in that** the first proportion quantity comprises or is an average value, formed over a plurality of breaths in the first supply phase, of the first proportion of the metabolically inert gas in the first inspiratory or expiratory working gas,
and/or
**in that** the second proportion quantity comprises or is an average value, formed over a plurality of breaths in the second supply phase, of the second proportion of the metabolically inert gas in the second inspiratory or expiratory working gas.

**10.** The ventilator (10) as recited in Claim 9,

**characterized in that** the plurality of breaths, over which the first proportion quantity is ascertained as an average value, is closer to the start of the ascertainment period and/or of the second supply phase than to the start of the first supply phase,
and/or
the plurality of breaths, over which the second proportion quantity is ascertained as an average value, is closer to the end of the ascertainment period and/or of the second supply phase than to the start of the ascertainment period or the second supply phase.

**11.** The ventilator (10) as recited in one of the preceding claims,
**characterized in that** the ascertainment of the functional residual capacity occurs on the basis of a quotient of the corrected difference in amount and the difference in proportion.

**12.** The ventilator (10) as recited in one of the preceding claims,
**characterized in that** at the end of a plurality of breaths during the second supply phase at the end of an expiration phase, a respiratory pressure in the airway of the patient and/or in a proximal area of a ventilation line is the PEEP.

**13.** The ventilator (10) as recited in one of the preceding claims,
**characterized in that** the ventilator (10) sensorially detects both an inspiratory respiratory gas flow as well as an expiratory respiratory gas flow.

**14.** The ventilator (10) as recited in one of the preceding claims,
**characterized in that** the ventilator (10) is configured to carry the method for ascertaining a functional residual capacity (FRC) of a lung of a patient during an artificial respiration of a patient.


**Revendications**

**1.** Dispositif de ventilation (10) conçu à la fois pour la ventilation artificielle au moins partielle de patients vivants (P) et pour la mise en œuvre d'un procédé de détermination d'une capacité résiduelle fonctionnelle (FRC) d'un poumon de patient, le procédé de détermination d'une capacité résiduelle fonctionnelle (FRC) d'un poumon de patient comprenant les caractéristiques suivantes :

- fourniture d'un premier gaz respiratoire inspiratoire contenant une première proportion d'un gaz métaboliquement inerte pendant une première phase d'alimentation temporelle (70),
- après la première phase d'alimentation (70) : fourniture d'un deuxième gaz respiratoire inspiratoire différent du premier, contenant une deuxième proportion différente de la première du gaz métaboliquement inerte pendant une deuxième phase d'alimentation temporelle (78),
- détermination d'une différence de quantité apparaissant pendant la deuxième phase d'alimentation (78), qui représente pour une période de détermination une quantité différentielle entre une quantité de gaz métaboliquement inerte inspiratoire et une quantité de gaz métaboliquement inerte expiratoire, la période de détermination ne se terminant pas après la deuxième phase d'alimentation,
- déterminer la capacité fonctionnelle résiduelle (FRC) en tenant compte de la différence de quantité et d'une différence de proportion entre une première grandeur de proportion, qui représente la première proportion du gaz métaboliquement inerte dans le premier gaz de travail inspiratoire, et une deuxième grandeur de proportion, qui représente la deuxième proportion du gaz métaboliquement inerte dans le deuxième gaz de travail inspiratoire,
- Détermination d'une différence de base (74) qui représente une différence entre une quantité tidale de gaz métaboliquement inerte inspiratoire et une quantité tidale de gaz métaboliquement inerte expiratoire dans la première et/ou la deuxième phase d'alimentation (78),

la détermination de la capacité résiduelle fonctionnelle (FRC) étant effectuée sur la base d'une différence de quantité corrigée et de la différence de proportion, la différence de quantité corrigée étant formée en tenant compte de la différence de base lors de la détermination de la différence de quantité,

le dispositif de ventilation (10) comprenant :

- une première source de gaz respiratoire (12) qui fournit un premier composant de gaz respiratoire inspiratoire (A1) avec une première fraction du gaz métaboliquement inerte,
- une deuxième source de gaz respiratoire (15) qui fournit un deuxième composant de gaz respiratoire inspiratoire (A2) avec une deuxième fraction du gaz métaboliquement inerte différente de la première,
- un dispositif de mélange réglable de manière variable (17) pour former un gaz respiratoire inspiratoire avec une proportion variable de gaz métaboliquement inerte à partir du premier et/ou du deuxième composant de gaz respiratoire inspiratoire (A1, A2),
- un agencement de conduits de ventilation (20) pour acheminer le gaz respiratoire inspiratoire vers une sortie de gaz respiratoire côté patient (62) et pour évacuer le gaz respiratoire expiratoire d'une entrée de gaz respiratoire côté patient (62),
- un ensemble de soupapes de commande comprenant une soupape inspiratoire (19in) et une soupape expiratoire (19ex),
- un dispositif de modification de pression (13) pour modifier au moins le gaz respiratoire inspiratoire dans l'ensemble de conduits de ventilation (20),
- un agencement de capteur de flux (48) pour détecter au moins le débit de gaz respiratoire inspiratoire,
- un agencement de capteurs de composants gazeux (52, 54) pour détecter directement ou indirectement la proportion de gaz métaboliquement inerte dans le gaz respiratoire inspiratoire et expiratoire,
- un dispositif de commande (14) qui est conçu pour commander le dispositif de soupape de commande et le dispositif de modification de pression (13) et qui est relié, en termes de transmission de signaux, à l'agencement de capteurs de flux (48) et à l'agencement de capteurs de composants gazeux (52, 54) pour transmettre les signaux de détection respectifs au dispositif de commande (14).

2.  Dispositif de ventilation (10) selon la revendication 1,
**caractérisé en ce que** l'agencement de capteurs de composants gazeux (52, 54) comprend au moins l'un des capteurs suivants :

- un capteur d'oxygène pour détecter la teneur en oxygène dans le gaz respiratoire inspiratoire et expiratoire, et
- un capteur de dioxyde de carbone pour détecter la teneur en dioxyde de carbone dans le gaz respiratoire inspiratoire et expiratoire.

3.  Dispositif de ventilation selon la revendication 1 ou 2,
**caractérisé en ce que** l'agencement de capteurs de composants gazeux (52, 54) est disposé dans une section de flux principal (64) de l'agencement de conduits de ventilation (20) pour détecter la proportion de gaz métaboliquement inerte dans le gaz respiratoire inspiratoire et expiratoire, qui est traversée à la fois par le gaz respiratoire inspiratoire fourni au patient (P) et par le gaz respiratoire expiratoire s'écoulant du patient (P).

4.  Dispositif de ventilation (10) selon l'une des revendications 1 à 3,
**caractérisé en ce que** le dispositif de commande (14) destiné à commander le dispositif de mélange (17) est conçu pour modifier la proportion de gaz métaboliquement inerte dans le gaz respiratoire inspiratoire en commandant le dispositif de mélange (17).

5.  Dispositif de ventilation (10) selon l'une des revendications précédentes,
**caractérisé en ce que** la différence de base (74) comprend au moins une valeur moyenne d'une pluralité de différences de volume courant entre respectivement un volume courant de gaz inspiratoire métaboliquement inerte et un volume courant de gaz expiratoire métaboliquement inerte pour une pluralité de cours respiratoires dans la première et/ou la deuxième phase d'alimentation (78).

6.  Dispositif de ventilation (10) selon l'une des revendications précédentes,

**caractérisé en ce que** la différence de base (74) comprend une valeur moyenne d'une pluralité de différences de volume courant entre respectivement un volume courant de gaz métaboliquement inerte inspiratoire et un volume courant de gaz métaboliquement inerte expiratoire pour une pluralité de courses respiratoires dans une section de détection initiale temporelle (82) située dans la première phase d'alimentation (70), la section de détection initiale (82) étant plus proche du début de la deuxième phase d'alimentation (78) que du début de la première phase d'alimentation (70),

et/ou

que la différence de base (74) comprend une valeur moyenne d'une pluralité de différences de volume courant entre respectivement un volume courant de gaz métaboliquement inerte inspiratoire et un volume courant de gaz métaboliquement inerte expiratoire pour une pluralité de courses respiratoires dans une section de détection finale temporelle (81) située dans la deuxième phase d'alimentation (78), la section de détection finale (81) étant plus proche de la fin de la deuxième phase d'alimentation (78) que de son début.

7. Dispositif de ventilation (10) selon l'une des revendications précédentes,
**caractérisé en ce que** la différence de base (74) comprend, au moins pendant une partie de la deuxième phase d'alimentation (78) et/ou pendant la période de détection (79), une différence de base tidale (74) qui est déterminée pour un course respiratoire en fonction d'une proportion du gaz métaboliquement inerte dans le gaz respiratoire du course respiratoire respectif.

8. Dispositif de ventilation (10) selon l'une des revendications précédentes,
**caractérisé en ce que** la différence de quantité corrigée correspond à une somme de différences de quantité tidale corrigées sur un certain nombre de courses respiratoires pendant la période de détermination, le dispositif de ventilation (10) étant conçu pour former pour chaque course respiratoire une différence de volume courant corrigée à partir d'une différence entre une différence de volume courant de ce course respiratoire et une différence de base associée au course respiratoire, le dispositif de ventilation (10) étant conçu pour former la différence de volume courant pour chaque course respiratoire à partir de la différence entre un volume courant de gaz inspiratoire métaboliquement inerte et un volume courant de gaz inspiratoire métaboliquement inerte de cette course respiratoire.

9. Dispositif de ventilation (10) selon l'une des revendications précédentes,

**caractérisé en ce que** la première grandeur proportionnelle comprend ou est une valeur moyenne de la première proportion du gaz métaboliquement inerte dans le premier gaz de travail inspiratoire ou expiratoire, formée sur une pluralité de courses respiratoires dans la première phase d'alimentation,
ou/et
que la deuxième grandeur proportionnelle comprend ou est une valeur moyenne de la deuxième proportion du gaz métaboliquement inerte dans le deuxième gaz de travail inspiratoire ou expiratoire, formée sur une pluralité de courses respiratoires dans la deuxième phase d'alimentation.

10. Dispositif de ventilation (10) selon la revendication 9,

**caractérisé en ce que** la pluralité de courses respiratoires sur lesquelles la première proportion est déterminée en tant que valeur moyenne est située plus près du début de la période de détermination et/ou de la deuxième phase d'alimentation que du début de la première phase d'alimentation,
et/ou
la pluralité des courses respiratoires sur lesquelles la deuxième grandeur proportionnelle est déterminée en tant que valeur moyenne est située plus près de la fin de la période de détermination et/ou de la deuxième phase d'alimentation que du début de la période de détermination ou de la deuxième phase d'alimentation.

11. Dispositif de ventilation (10) selon l'une des revendications précédentes,
**caractérisé en ce que** la détermination de la capacité résiduelle fonctionnelle s'effectue sur la base d'un quotient de la différence de quantité corrigée et de la différence de proportion.

12. Dispositif de ventilation (10) selon l'une des revendications précédentes,
**caractérisé en ce qu'**à la fin d'une pluralité de courses respiratoires pendant la deuxième phase d'alimentation, à la fin d'une phase d'expiration, une pression respiratoire dans les voies respiratoires du patient et/ou dans une zone proximale d'un conduit de ventilation est la PEEP.

13. Dispositif de ventilation (10) selon l'une des revendications précédentes,
**caractérisé en ce que** le dispositif de ventilation (10) détecte à la fois un flux de gaz respiratoire inspiratoire et un flux de gaz respiratoire expiratoire.

**14.** Dispositif de ventilation (10) selon l'une des revendications précédentes,
**caractérisé en ce que** le dispositif de ventilation (10) est conçu pour mettre en œuvre le procédé de détermination d'une capacité résiduelle fonctionnelle (FRC) d'un poumon de patient pendant une ventilation artificielle d'un patient.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7530353 B2

- WO 0044280 A1

**In der Beschreibung aufgeführte Nicht-Patentliteratur**

- **OLEGÅRD, C. et al.** Estimation of Functional Residual Capacity at the Bedside Using Standard Monitoring Equipment: A Modified Nitrogen Washout/Washin Technique Requiring a Small Change of the Inspired Oxygen Fraction. *International Anesthesia Research Society: "Anesthesia & Analgesia*, 2005 (101), 206-212

- FRC-Messung bei beatmeten Intensivpatienten - Eine Standortbestimmung. **WAUER, H. J. et al.** Der Anaesthesist. Springer Verlag, 1998, 844-855